# EUROPÄISCHE PATENTSCHRIFT

(11) **EP 3 764 950 B1**
(45) Veröffentlichungstag und Bekanntmachung des Hinweises auf die Patenterteilung: **29.12.2021**
(21) Anmeldenummer: 19710670.1
(22) Anmeldetag: 12.03.2019
(51) Int. Cl.: A61F 2/32, A61F 2/36, A61F 2/30

(54) **IMPLANTAT**
IMPLANT
IMPLANT

(30) Priorität: 12.03.2018 DE 102018105682
(43) Veröffentlichungstag der Anmeldung: 20.01.2021
(73) Patentinhaber: Aesculap AG, 78532 Tuttlingen (DE)
(72) Erfinder: SAUERESSIG, Thomas, 78532 Tuttlingen (DE)
(74) Vertreter: Hoeger, Stellrecht & Partner Patentanwälte mbB
(86) Internationale Anmeldenummer: PCT/EP2019/056082
(87) Internationale Veröffentlichungsnummer: WO 2019/175136

(56) Entgegenhaltungen:
- WO-A1-2017/098034
- DE-C1- 19 926 391
- US-A- 4 176 582

## Beschreibung

Die Erfindung ist in den beigefügten Ansprüchen definiert. Hier offenbarte Aspekte, Ausführungsformen und Beispiele, die nicht in den Umfang der beigefügten Ansprüche fallen, sind nicht Teil der Erfindung und werden lediglich zu Veranschaulichungszwecken bereitgestellt.

Die vorliegende Erfindung betrifft ein Implantat mit einem ersten Implantatteil und einem zweiten Implantatteil, wobei der erste und der zweite Implantatteil über eine eine Mutter umfassende Schraubverbindung in einer Verbindungsstellung miteinander verbunden sind, wobei die Mutter in Form einer medizinischen Mutter ausgebildet ist, welche eine Längsachse definiert und ein Innengewinde und einen Außenmehrkant aufweist.

Ferner werden nachfolgend medizinische Muttern beschrieben, welche eine Längsachse definieren und ein Innengewinde und einen Außenmehrkant aufweisen.

Implantate, welche mehr als zwei Implantatteile umfassen, werden auch als modulare Implantate bezeichnet. Um die Implantatteile des modularen Implantats miteinander zu verbinden, ist es bekannt, Schraubverbindungen zu nutzen. Beispielsweise kann an einem ersten Implantatteil ein mit einem Außengewinde versehener Bolzenabschnitt ausgebildet sein, welcher eine Durchbrechung eines zweiten Implantatteils durchsetzt. So ist es möglich, eine Mutter auf den mit einem Außengewinde versehenen Bolzenabschnitt, welcher durch die Durchbrechung des zweiten Implantatteils vorsteht, aufzuschrauben. Auf diese Weise lassen sich die zwei Implantatteile gegeneinander verspannen. Ein Problem bei derartigen Schraubverbindungen, die insbesondere bei Revisionsimplantaten zum Einsatz kommen, ist es, dass ein Anzugsdrehmoment zum Anziehen der Mutter begrenzt werden muss, um eine Beschädigung der Schraubverbindungen zu vermeiden. Um dies sicherzustellen, werden Drehmomentschlüssel zum Anziehen der Mutter verwendet.

Insbesondere dann, wenn bei einem chirurgischen Eingriff mehrere Muttern mit unterschiedlichen Drehmomenten angezogen werden müssen, müssen entweder mehrere Drehmomentschlüssel bereitgestellt werden oder ein Drehmomentschlüssel, der es ermöglicht, das Anzugsdrehmoment individuell einzustellen. In jedem Fall ist es jedoch möglich, dass es zu Verwechslungen kommt und eine Mutter mit einem zu großen Anzugsdrehmoment angezogen wird. Im ungünstigsten Fall kann dies zu einer Beschädigung der Schraubverbindung und damit des Implantats insgesamt führen. Insbesondere kann es sofort oder auch zu einem späteren Zeitpunkt zu einem Bruch des Implantats im Bereich der Schraubverbindung kommen.

Ein Implantat der eingangs beschriebenen Art ist in der WO 2017/098034 A1 offenbart. Aus der US 4,176,582 ist eine Muttervorrichtung zur Begrenzung eines Drehmoments bekannt. In der EP 0 361 111 A2 ist eine Lichtwellenleiter-Steckverbindung beschrieben. Ein Prothesenschaft ist in der DE 199 26 391 C1 offenbart.

Es ist daher eine Aufgabe der vorliegenden Erfindung, ein Implantat der eingangs beschriebenen Art so zu verbessern, dass die Mutter einfach und sicher mit einem vorgegebenen Anzugsdrehmoment angezogen werden kann.

Diese Aufgabe wird bei einem Implantat der eingangs beschriebenen Art erfindungsgemäß dadurch gelöst, dass die Mutter mindestens ein Innenteil und mindestens ein das Innenteil mindestens teilweise, insbesondere vollständig oder im Wesentlichen vollständig, umgebendes Außenteil umfasst, dass das mindestens eine Innenteil das Innengewinde umfasst, dass das mindestens eine Außenteil den Außenmehrkant umfasst, dass die Mutter eine Drehmomentbegrenzungseinrichtung umfasst, welche derart ausgebildet ist, dass bei einem in einer Anzugsdrehrichtung auf das mindestens eine Außenteil wirkenden Drehmoment, welches kleiner als ein Grenzdrehmoment ist, das mindestens eine Außenteil und das mindestens eine Innenteil in der Anzugsdrehrichtung relativ zueinander unverdrehbar sind, dass bei einem in der Anzugsdrehrichtung auf das mindestens eine Außenteil wirkenden Drehmoment, welches mindestens dem Grenzdrehmoment entspricht, das mindestens eine Außenteil und das mindestens eine Innenteil in der Anzugsdrehrichtung relativ zueinander verdrehbar sind, dass das mindestens eine Innenteil und das mindestens eine Außenteil unlösbar miteinander gekoppelt sind, dass die medizinische Mutter zweiteilig ausgebildet ist und ausschließlich ein einziges Innenteil und ein einziges Außenteil umfasst oder dass die medizinische Mutter dreiteilig ausgebildet ist und ausschließlich ein einziges Innenteil und zwei Außenteile umfasst.

Ein Implantat mit einer derart weitergebildeten medizinischen Mutter ermöglicht es, auf einen Drehmomentschlüssel zu verzichten. Eine Drehmomentbegrenzung wird hier durch die Mutter selbst erreicht, die eine Drehmomentbegrenzungseinrichtung umfasst. Auf diese Weise können Verwechslungen bei Anzugsdrehmomenten bei unterschiedlichen Muttern und/oder mit unterschiedlichen Drehmomentschlüsseln auf einfache Weise vermieden werden. Jede bei beispielsweise einer Implantation eines Implantats verwendete Mutter zur Verschraubung zweier Implantatteile miteinander kann so auf einfache Weise mit einem herkömmlichen Maul- oder Ringschlüssel angezogen werden. Das Überschreiten eines Anzugsdrehmoments ist nicht möglich und wird durch die Mutter selbst, nämlich durch deren Drehmomentbegrenzungseinrichtung, verhindert. Der Außenmehrkant der Mutter kann insbesondere in Form eines Drei-, Vier-, Fünf-, Sechs-, Sieben- oder Achtkant ausgebildet sein. Alternativ kann der Außenmehrkant auch in Form eines Außenvielrund ausgebildet sein. Eine medizinische Mutter zeichnet sich insbesondere dadurch aus, dass sie sterilisierbar ist. Mit anderen Worten ist sie vorzugsweise aus einem oder mehreren sterilisierbaren Materialien ausgebildet, so dass sie vor einer Implantation sterilisiert und dann steril in den Körper eines Menschen oder eines Tieres implantiert werden kann. Insbesondere können das oder die Materialien, aus denen die medizinische Mutter ausgebildet ist, heißdampf-sterilisierbar oder gamma-sterilisierbar sein. Ferner ist es vorteilhaft, dass das mindestens eine Innenteil und das mindestens eine Außenteil unlösbar miteinander gekoppelt sind. Dies ermöglicht es insbesondere, die medizinische Mutter wie eine herkömmliche, einstückig oder monolithisch ausgebildete Mutter zu nutzen. Insbesondere können das mindestens eine Innenteil und das mindestens eine Außenteil unverlierbar aneinander gesichert sein, wobei jedoch stets die zum Anziehen und Lösen der Mutter erforderlichen Relativbewegungen zwischen dem mindestens einen Innenteil und dem mindestens einen Außenteil möglich sind. Eine unlösbare Kopplung kann insbesondere durch Verstemmen von Bünden, insbesondere freier Enden an Innen- und/oder Außenteilen erreicht werden. Durch ein solches Verstemmen kann ein Lösen miteinander gekoppelter Teile der Mutter voneinander nur durch nicht bestimmungsgemäße Umformung verstemmter Abschnitte von Innen- und/oder Außenteilen erreicht werden. Dabei wird die Mutter jedoch beschädigt und damit unbrauchbar. Gemäß der Erfindung ist die medizinische Mutter zweiteilig ausgebildet und umfasst ausschließlich ein einziges Innenteil und ein einziges Außenteil oder dass die medizinische Mutter dreiteilig ausgebildet ist und ausschließlich ein einziges Innenteil und zwei Außenteile umfasst. Das einzige Innenteil und das einzige Außenteil können insbesondere einstückig oder einteilig ausgebildet sein. So kann die medizinische Mutter mit Drehmomentbegrenzungseinrichtung aus nur zwei Elementen ausgebildet werden. Dies vereinfacht die Herstellung sowie auch die damit verbundenen Kosten. Die dreiteilige Mutter umfasst ein einziges Innenteil mit einer Innenteilverzahnung und zwei Außenteile, von denen jedoch nur eines eine Außenteilverzahnung aufweist. Ein zweites Außenteil kann insbesondere in Form eines Klemmrings zum Anlegen an eines der beiden Implantatteile ausgebildet und relativ zum Innenteil axial, also parallel zur Längsachse, unbeweglich oder im Wesentlichen unbeweglich, jedoch um die Längsachse verdrehbar angeordnet sind. Der Klemmring ist vorzugsweise ohne eine Außenteilverzahnung ausgebildet, was eine einfache Verdrehbarkeit von Klemmring und Innenteil relativ zueinander ermöglicht.

Auf besonders einfache und kompakte Weise ausbilden lässt sich die medizinische Muttern, wenn das mindestens Außenteil und/oder das mindestens eine Innenteil temporär verformbar ausgebildet sind, so dass sich das mindestens Außenteil und/oder das mindestens eine Innenteil bei einem in der Anzugsdrehrichtung auf das mindestens eine Außenteil wirkenden Drehmoment, welches mindestens dem Grenzdrehmoment entspricht, derart verformen, dass das mindestens eine Außenteil und das mindestens eine Innenteil in der Anzugsdrehrichtung relativ zueinander verdrehbar sind. Insbesondere kann sich das mindestens eine Außenteil insgesamt verformen, beispielsweise durch Verformung eines hülsenförmigen Wandabschnitts desselben, welcher von der Drehmomentbegrenzungseinrichtung umfasst ist und mit einem korrespondierenden Innenteilabschnitt des mindestens einen Innenteils zusammenwirkt. Durch die verformbare Ausbildung des Außenteils ist es insbesondere möglich, auf zusätzliche Federn zur Ausbildung der Drehmomentbegrenzungseinrichtung zu verzichten, wodurch sich der Aufbau der Mutter deutlich vereinfacht. Ferner kann das mindestens eine Innenteil insbesondere derart ausgebildet sein, dass es sich verformen kann, und zwar beispielsweise so, dass sich eine Länge desselben in einer Richtung parallel zur Längsachse ändert. Diese Ausgestaltung kann insbesondere vorgesehen sein, wenn, wie nachfolgend noch beschrieben, die Drehmomentbegrenzungseinrichtung eine am mindestens einen Innenteil angeordnete oder ausgebildete, in axialer Richtung weisende Innenteilverzahnung und eine am mindestens einen Außenteil angeordnete oder ausgebildete, in axialer Richtung auf die Innenteilverzahnung weisende und mit der Innenteilverzahnung zusammenwirkende Außenteilverzahnung umfasst.

Gemäß einer bevorzugten Ausführungsform der Erfindung kann vorgesehen sein, dass die Drehmomentbegrenzungseinrichtung eine am mindestens einen Innenteil angeordnete oder ausgebildete, von der Längsachse in radialer Richtung weg weisende oder in axialer Richtung weisende Innenteilverzahnung und eine am mindestens einen Außenteil angeordnete oder ausgebildete, in radialer Richtung auf die Längsachse hin weisende oder in axialer Richtung auf die Innenteilverzahnung weisende und mit der Innenteilverzahnung zusammenwirkende Außenteilverzahnung umfasst derart, dass bei einem in der Anzugsdrehrichtung auf das mindestens eine Außenteil wirkenden Drehmoment, welches kleiner als das Grenzdrehmoment ist, das mindestens eine Außenteil und das mindestens eine Innenteil in der Anzugsdrehrichtung relativ zueinander unverdrehbar sind und dass bei einem in der Anzugsdrehrichtung auf das mindestens eine Außenteil wirkenden Drehmoment, welches mindestens dem Grenzdrehmoment entspricht, das mindestens eine Außenteil und das mindestens eine Innenteil in der Anzugsdrehrichtung relativ zueinander verdrehbar sind. Die Innenteilverzahnung und die Außenteilverzahnung sind vorzugsweise derart angeordnet und bemessen, dass die Verdrehbarkeit zwischen dem mindestens einen Außenteil und dem mindestens einen Innenteil wie angegeben erfolgen. Insbesondere können sie insbesondere einstückig mit oder unbeweglich am am mindestens einen Innenteil beziehungsweise am mindestens einen Außenteil angeordnet oder ausgebildet sein. Insbesondere können die Innenteilverzahnung und die Außenteilverzahnung ausgebildet sein durch eine über einen Umfang des mindestens einen Innenteils beziehungsweise des mindestens einen Außenteils variierende Dicke derselben. So kann insbesondere eine Stabilität der medizinischen Mutter erhöht werden, da keine federnden Laschen oder dergleichen ausgebildet werden müssen. Zudem lässt sich so auf einfache Weise jeweils eine einstückige Ausbildung des mindestens einen Innenteils und des mindestens einen Außenteils realisieren. Insbesondere kann die medizinische Mutter so aus nur zwei Teilen ausgebildet werden, nämlich einem einzigen Innenteil und einem einzigen Außenteil. Insbesondere können der mindestens eine Innengewindeabschnitt und ein Abschnitt des mindestens einen Innenteils, an dem die Innenteilverzahnung ausgebildet ist, axial zueinander versetzt angeordnet sein. Alternativ können sie in axialer Richtung auch überlappend angeordnet oder ausgebildet sein. Insbesondere kann es vorteilhaft sein, wenn die Innenteilverzahnung und die Außenteilverzahnung in in axialer Richtung weisen, das mindestens eine Innenteil und/oder das mindestens eine Außenteil derart ausgebildet sind, dass sich mindestens eines davon verformen kann, und zwar beispielsweise so, dass sich eine Länge desselben in einer Richtung parallel zur Längsachse ändert.

Günstig ist es, wenn das mindestens eine Innenteil und das mindestens eine Außenteil in einer Löserichtung derart zusammenwirken, dass bei einer Verdrehung des mindestens einen Außenteils um die Längsachse in einer der Anzugsdrehrichtung entgegengesetzt gerichteten Lösedrehrichtung das mindestens eine Innenteil zusammen mit dem mindestens einen Außenteil in der Lösedrehrichtung verdreht wird. Diese Ausgestaltung ermöglicht auf einfache Weise ein Lösen der medizinischen Mutter, um beispielsweise eine Verschraubung zu lösen zum Trennen von zwei durch die Verschraubung verbundenen Implantatteilen. Beispielsweise kann dies erreicht werden über eine Mitnehmereinrichtung mit mindestens einem Mitnehmer, welcher in Folge einer Rotation des mindestens einen Außenteils in der Lösedrehrichtung das mindestens eine Innenteil mitnimmt, also zwangsverdreht.

Auf besonders einfache Weise kann die medizinische Mutter gelöst werden, wenn die Innenteilverzahnung und die Außenteilverzahnung bei Verdrehung des mindestens einen Außenteils um die Längsachse in der Lösedrehrichtung ineinandergreifen. Die Innenteilverzahnung und die Außenteilverzahnung können hierfür insbesondere unidirektional ausgebildet sein, so dass ein Ineinandergreifen derselben nur bei Verdrehung des mindestens einen Außenteils um die Längsachse in der Lösedrehrichtung möglich ist.

Vorteilhaft ist es, wenn die Innenteilverzahnung eine sich in distaler Richtung erweiternde konische Außenkontur definiert. Eine solche konische Außenkontur hat insbesondere den Vorteil, dass die Innenteilverzahnung bei einer konischen Aufweitung des Außenteils aufgrund einer Verdrehung von Innenteil und Außenteil in der Anzugsdrehrichtung besser an das Außenteil angepasst ist. Zahnflanken von Zähnen der zusammenwirkenden Innenteil- und Außenteilverzahnung können so gleichmäßig und im Wesentlichen über ihre gesamte Länge parallel zur Längsachse aneinander anliegen, so dass sich keine punktuelle Kraftübertragung zwischen Außenteil und Innenteil ergibt, sondern mindestens eine linienhafte, eher flächige Anlage und Kraftübertragung. Insbesondere kann ein von der Außenkontur definierter Konuswinkel in einem Bereich von etwa 0° bis etwa 5° liegen. Vorzugsweise beträgt der Konuswinkel 1,5°.

Auf besonders einfache und kostengünstige Weise lässt sich die medizinische Mutter ausbilden, wenn das mindestens eine Außenteil in Form einer Außenhülse ausgebildet ist. Insbesondere kann die Außenhülse mehrere Außenhülsenabschnitte aufweisen, insbesondere zwei, drei oder vier, die sich in ihrem Außendurchmesser unterscheiden. Beispielsweise kann der Außenmehrkant an einem dieser Abschnitte ausgebildet sein. Ferner kann eine hülsenförmige Ausgestaltung des mindestens einen Außenteils, insbesondere bei entsprechend vorgesehenen Wandstärken unter Berücksichtigung eines Materials, aus dem das mindestens eine Außenteil ausgebildet ist, eine Verformung des mindestens einen Außenteils in einer ausreichenden Weise genau dann ermöglichen, wenn ein in der Anzugsdrehrichtung auf das mindestens eine Außenteil wirkendes Drehmoment dem Grenzdrehmoment entspricht oder größer ist. Eine gewünschte Verformung des mindestens einen Außenteils in Abhängigkeit eines auf das mindestens eine Außenteil einwirkenden Drehmoments kann insbesondere dadurch erreicht werden, dass das mindestens eine Außenteil eine Außenteilwand aufweist und das eine Dicke der Außenteilwand über einen Umfang des mindestens einen Außenteils variiert. Insbesondere kann eine Dicke der Außenteilwand abschnittsweise derart verringert sein, dass in den Bereichen mit einer verringerten Dicke der Außenteilwand eine definierte Verformung bei einem hinreichend großen, auf das mindestens eine Außenteil wirkenden Drehmoments möglich ist. So lassen sich auf einfache Weise definiert verformbare Abschnitte über den Umfang der Außenteilwand ausbilden, die sich zudem parallel zur Längsachse erstrecken können. Insbesondere kann eine Dicke bereichsweise auch auf Null reduziert werden, wenn zum Beispiel eine Durchbrechung vorgesehen ist.

Günstig ist es, wenn das mindestens eine Außenteil in der Außenteilwand mindestens einen Schlitz aufweist. Im Bereich des Schlitzes wird das mindestens eine Außenteil geschwächt, so dass es sich leichter verformen lässt. Durch Größe und Position des Schlitzes an der Außenteilwand kann so eine Verformung des mindestens einen Außenteils aufgrund eines einwirkenden Drehmoments definiert vorgegeben werden.

Um die Verformbarkeit des mindestens einen Außenteils weiter zu verbessern und insbesondere gezielt einstellen zu können, ist es vorteilhaft, wenn die medizinische Mutter eine Mehrzahl von Schlitzen umfasst. Insbesondere können die Schlitze parallel zueinander ausgerichtet sein. Ein Abstand zwischen benachbarten Schlitzen ist vorzugsweise größer als eine Breite der Schlitze.

Um insbesondere eine Querschnittsform des mindestens einen Außenteils verändern zu können, ist es günstig, wenn der mindestens eine Schlitz parallel zur Längsachse verläuft. Wenn eine Verformbarkeit insbesondere auch in einer Richtung parallel zur Längsachse erreicht werden soll, ist es günstig wenn der mindestens eine Schlitz schräg zur Längsachse verläuft. Derartige Schlitze erstrecken sich somit mit einer Komponente in Umfangsrichtung am mindestens einen Außenteil. Der mindestens eine Schlitz kann insbesondere in Form einer Durchbrechung der Außenteilwand ausgebildet sein. Vorzugsweise ist eine Länge des mindestens einen Schlitzes größer als eine Breite desselben. Beispielsweise kann eine Länge des Schlitzes mindestens drei, insbesondere fünfmal so groß sein wie dessen Breite.

Vorteilhafterweise ist das mindestens eine Innenteil in Form einer Innenhülse ausgebildet. Die Innenhülse kann insbesondere eine Durchbrechung parallel zur Längsachse aufweisen. Ferner kann die Innenhülse auch mehrere Abschnitte aufweisen, die einen unterschiedlichen Außen- und/oder Innendurchmesser definieren. Insbesondere kann eine von der Innenhülse definierte Außenkontur im Bereich der Innenteilverzahnung größer sein als im Bereich eines daran angrenzenden Abschnitts. Insbesondere kann ein Außendurchmesser eines mit dem Innengewinde versehenen Abschnitts der Innenhülse parallel zur Längsachse kleiner sein als im Bereich der Innenteilverzahnung. Das Innengewinde kann sich über die gesamte oder auch nur einen Teil einer Länge der Innenhülse erstrecken.

Günstig ist es, wenn das mindestens eine Außenteil und das mindestens eine Innenteil in der Löserichtung um die Längsachse um einen Lösedrehwinkel von höchstens 360° relativ zueinander verdrehbar sind. Der Lösedrehwinkel kann insbesondere durch einen Abstand von zwei Zähnen der Innenteilverzahnung oder von zwei Zähnen der Außenteilverzahnung vorgegeben werden. Je mehr Zähne die Innenteilverzahnung beziehungsweise die Außenteilverzahnung umfassen, umso kleiner kann der Lösedrehwinkel vorgegeben werden.

Ferner kann es vorteilhaft sein, wenn das mindestens eine Außenteil und das mindestens eine Innenteil in der Anzugsdrehrichtung um die Längsachse um einen Anzugsdrehwinkel von höchstens 360° relativ zueinander verdrehbar sind, wenn das Anzugsdrehmoment kleiner als das Grenzdrehmoment ist. Auch hier kann der Anzugsdrehwinkel beispielsweise definiert werden durch einen Abstand von zwei benachbarten Zähnen der Innenteilverzahnung beziehungsweise der Außenteilverzahnung. Ist das Anzugsdrehmoment größer als das Grenzdrehmoment, ist eine Verdrehung um mehr als 360° möglich. Vorzugsweise steht das mindestens eine Außenteil distalseitig über das mindestens eine Innenteil vor. Insbesondere dann, wenn die Innenteilverzahnung und die Außenteilverzahnung distalseitig am mindestens einen Außenteil beziehungsweise am mindestens einen Innenteil angeordnet oder ausgebildet sind, kann so dass das mindestens eine Außenteil das mindestens eine Innenteil und die miteinander zusammenwirkenden Innenteil- und Außenteilverzahnungen schützen.

Günstigerweise steht das mindestens eine Innenteil proximalseitig über das mindestens eine Außenteil vor. Diese Ausgestaltung ermöglicht es insbesondere, das mindestens eine Innenteil und das mindestens eine Außenteil aneinander in axialer Richtung, also parallel zur Längsachse aneinander zu sichern, wenn zum Beispiel ein proximaler Rand des Innenteils in radialer Richtung nach außen verstemmt wird. Eine solche Kopplung zwischen dem mindestens einen Innenteil und dem mindestens einen Außenteil auszubilden ist insbesondere dann vorteilhaft, wenn das mindestens eine Außenteil am mindestens einen Innenteil gegen eine Bewegung in distaler Richtung gesichert ist, beispielsweise durch einen Anschlag am mindestens einen Innenteil, welcher in proximaler Richtung wirkt. Ein solcher Anschlag kann beispielsweise durch eine Veränderung eines Querschnitts am mindestens einen Innenteil ausgebildet werden, welcher mit einem korrespondierenden Anschlag am mindestens einen Außenteil, welcher in distaler Richtung, zusammenwirkt.

Gemäß einer weiteren bevorzugten Ausführungsform der Erfindung kann vorgesehen sein, dass die Innenteilverzahnung mindestens einen Innenteilzahn umfasst und dass die Außenteilverzahnung mindestens einen Außenteil umfasst. Insbesondere kann die Innenteilverzahnung einen, zwei, drei, vier, fünf, sechs, sieben, acht oder neun Innenteilzähne umfassen. Ferner kann die Außenteilverzahnung insbesondere einen, zwei, drei, vier, fünf, sechs, sieben, acht oder neun Außenteilzähne umfassen. Wie bereits oben dargelegt, kann über einen Abstand zwischen zwei benachbarten Zähnen der Innenteilverzahnung beziehungsweise der Außenteilverzahnung ein Lösedrehwinkel beziehungsweise ein Anzugsdrehwinkel vorgegeben werden. Ferner können Anzugsdrehmomente schonender vom mindestens einen Außenteil auf das mindestens eine Innenteil übertragen werden, je mehr Zähne die Innenteilverzahnung beziehungsweise die Außenteilverzahnung umfassen. Insbesondere muss die Anzahl der Zähne der Innenteilverzahnung und der Außenteilverzahnung nicht identisch sein. Für eine möglichst gleichmäßige Verformung des mindestens einen Außenteils ist es jedoch vorteilhaft, wenn die Anzahl der Innenteilzähne der Anzahl der Außenteilzähne entspricht und wenn diese derart angeordnet sind, dass sie alle gleichzeitig ineinandergreifen.

Vorzugsweise entspricht eine Anzahl der Schlitze in der Außenteilwand einer Anzahl der Außenteilzähne. So kann insbesondere ein Bereich der Außenteilwand und damit des mindestens einen Außenteils zwischen zwei Außenteilzähnen durch Ausbildung eines dazwischen angeordneten Schlitzes in definierter Weise elastisch ausgebildet werden, so dass ein Einleiten eines Anzugsdrehmoments auf das mindestens eine Außenteil, welches größer als das Grenzdrehmoment ist, zu einer Verformung des mindestens einen Außenteils führt, mit der Folge, dass das Außenteil relativ zum mindestens einen Innenteil uneingeschränkt verdrehbar ist.

Vorteilhaft ist es, wenn in der Außenteilwand Außenteilzähne und Schlitze gleichmäßig über einen Umfang verteilt, insbesondere alternierend, angeordnet oder ausgebildet sind. Wie bereits beschrieben können so Anzugsdrehmomente und Lösedrehmomente besonders gleichmäßig vom mindestens einen Außenteil auf das mindestens eine Innenteil übertragen werden.

Um eine Verformbarkeit des mindestens einen Außenteils in definierter Weise vorgeben zu können, ist es günstig, wenn die Dicke der Außenteilwand zwischen zwei Außenteilzähnen ein Minimum aufweist. Die Außenteilwand ist damit im Bereich zwischen zwei Außenteilzähnen leichter verformbar. Durch Vorgabe der Dicke der Außenteilwand im Bereich zwischen zwei Außenteilzähnen sowie unter Berücksichtigung des Materials, aus dem das mindestens eine Außenteil ausgebildet ist, kann so das Grenzdrehmoment für die medizinische Mutter in gewünschter Weise vorgegeben werden.

Vorteilhaft ist es, wenn sich der mindestens eine Innenteilzahn parallel oder im Wesentlichen parallel zur Längsachse erstreckt oder relativ zur Längsachse um einen Neigungswinkel geneigt ist. Insbesondere kann der Neigungswinkel einen Wert in einem Bereich von etwa 0° bis etwa 10° aufweisen.

Gemäß einer weiteren bevorzugten Ausführungsform der Erfindung kann vorgesehen sein, dass der mindestens eine Innenteilzahn mindestens eine Innenteilzahnlöseflanke aufweist, dass der mindestens eine Außenteilzahn mindestens eine Außenteilzahnlöseflanke aufweist und dass die Innenteilzahnlöseflanke und die Außenteilzahnlöseflanke in einer Lösestellung beim Verdrehen der Mutter in der Löserichtung aneinander anliegen. Der mindestens eine Innenteilzahn und der mindestens ein Außenteilzahn wirken also in der Lösestellung derart zusammen, dass sie praktisch gegenseitig Mitnehmer bilden, so dass ein Lösedrehmoment vom mindestens einen Außenteil auf das mindestens eine Innenteil zum Lösen der medizinischen Mutter übertragen werden kann.

Um eine sichere Funktion der medizinischen Mutter zu ermöglichen, ist es günstig, wenn die Innenteilzahnlöseflanke und/oder die Außenteilzahnlöseflanke eine die Längsachse enthaltende Schnittebene unter einem Winkel von maximal 30° schneiden. Insbesondere können so Innenteilzähne und Außenteilzähne mit und ohne Hinterschnitt ausgebildet werden.

Um insbesondere ein sicheres Lösen der medizinischen Mutter erreichen zu können, ist es günstig, wenn die mindestens eine Innenteilzahnlöseflanke bezogen auf eine die Längsachse und eine Innenteilzahnspitze des mindestens einen Innenteilzahns enthaltende Radialebene hinterschnitten ist und/oder wenn die mindestens eine Außenteilzahnlöseflanke bezogen auf eine die Längsachse und eine Außenteilzahnspitze des mindestens einen Außenteilzahns enthaltende Radialebene hinterschnitten ist. Die mindestens eine Innenteilzahnlöseflanke und die mindestens eine Außenteilzahnlöseflanke können so in der Lösestellung ineinandergreifen, wobei ein Herausrutschen der Zähne aus der Lösestellung aufgrund von deren hinterschnittener Ausgestaltung nicht möglich ist.

Ferner ist es vorteilhaft, wenn der mindestens eine Außenteilzahn mindestens eine Außenteilzahnaufgleitflanke aufweist, wenn der mindestens eine Innenteilzahn mindestens einen Innenteilzahnaufgleitflanke aufweist, wenn die mindestens einen Außenteilzahnaufgleitflanke und die mindestens eine Innenteilzahnaufgleitflanke aneinander klemmend gehalten sind, wenn das in der Anzugsdrehrichtung auf das mindestens eine Außenteil wirkende Drehmoment kleiner als das Grenzdrehmoment ist, und wenn die mindestens einen Außenteilzahnaufgleitflanke und die mindestens eine Innenteilzahnaufgleitflanke aneinander entlanggleiten, wenn das in der Anzugsdrehrichtung auf das mindestens eine Außenteil wirkende Drehmoment mindestens dem Grenzdrehmoment entspricht. Insbesondere können die mindestens eine Innenteilzahnaufgleitflanke und die mindestens Außenteilzahnaufgleitflanke bei einem Drehmoment, das mindestens dem Grenzdrehmoment entspricht, übereinander hinweg gleiten, so dass das mindestens eine Innenteil und das mindestens eine Außenteil ohne Beschränkung zueinander in der Anzugsdrehrichtung verdrehbar sind. Der mindestens eine Außenteilzahn kann ein, zwei, drei oder mehr Außenteilzahnaufgleitflanken aufweisen, welche insbesondere gegeneinander geneigte Flächen definieren können. In analoger Weise kann der mindestens eine Außenteilzahn auch eine, zwei, drei, vier oder mehr Außenteilzahnaufgleitflanken aufweisen, die ebenfalls gegeneinander geneigte Flächen definieren können. Die mindestens eine Außenteilzahnaufgleitflanke und die mindestens eine Innenteilzahnaufgleitflanke können ebene oder auch gekrümmte Flächen definieren, wobei die mindestens eine Innenteilzahnaufgleitflanke in Richtung auf das mindestens eine Außenteil hin weisend konvex gekrümmt sein kann. Ferner kann die mindestens eine Außenteilzahnaufgleitflanke in Richtung auf das mindestens eine Innenteil hin weisend auch konkav gekrümmt sein. Vorzugsweise definiert die mindestens eine Innenteilzahnaufgleitflanke, die die mindestens eine Innenteilzahnlöseflanke schneidet, diejenige Fläche, die bei Unterschreiten des Grenzdrehmoments klemmend am mindestens einen Außenteil gehalten ist. Insbesondere kann die Klemmung im Bereich der mindestens einen Außenteilzahnaufgleitflanke erfolgen, welche die Außenteilzahnlöseflanke schneidet.

Günstig ist es, wenn der mindestens eine Innenteilzahn (58) eine Innenteilzahnaußenfläche definiert, wenn die Innenteilzahnaußenfläche und die Innenteilzahnaufgleitfläche gegeneinander geneigt sind und in einem verrundeten Übergangsbereich ineinander übergehen. Ein solcher verrundeter Übergangsbereich, welcher insbesondere von der Längsachse weg weisend konvex gekrümmt sein kann, hat insbesondere den Vorteil, dass das Innenteil und das Außenteil bei einer Verdrehung relativ zueinander nicht blockieren können. Vorzugsweise liegt ein Radius des Übergangsbereichs in einem Bereich von etwa 0,5 mm bis etwa 3 mm liegt, insbesondere beträgt der Radius 1,5 mm.

Vorteilhaft ist es, wenn die mindestens eine Außenteilzahnaufgleitflanke flacher ist als die mindestens eine Außenteilzahnlöseflanke und/oder wenn die mindestens eine Innenteilzahnaufgleitflanke flacher ist als die mindestens eine Innenteilzahnlöseflanke. So lassen sich eine Drehmomentbegrenzung in der Anzugsdrehrichtung sowie das Lösen der Mutter durch Verdrehen des mindestens einen Außenteils und des mindestens einen Innenteils relativ zueinander in der Löserichtung einfach und sicher realisieren.

Günstig ist es, wenn die Anzahl der Innenteilzähne der Innenteilverzahnung der Anzahl der Außenteilzähne der Außenteilverzahnung entspricht oder kleiner ist oder größer ist. Je mehr Innenteilzähne und korrespondierende Außenteilzähne gleichzeitig miteinander zusammenwirken, umso sicherer können Anzugsdrehmomente und Lösedrehmomente vom mindestens einen Außenteil auf das mindestens eine Innenteil übertragen werden. Insbesondere ist es vorteilhaft, wenn ein Zahnverhältnis der Anzahl der Außenteilzähne und der Anzahl der Innenteilzähne in einem Bereich von etwa 0,7 bis etwa 1,3 liegt. Günstig ist es, wenn das Zahnverhältnis 1 oder im Wesentlichen 1 beträgt.

Vorteilhaft ist es, wenn das mindestens eine Innenteil eine sich koaxial zur Längsachse erstreckende Längsdurchbrechung aufweist und wenn die Längsdurchbrechung mindestens einen das Innengewinde bildenden Innengewindeabschnitt umfasst. Alternativ kann das mindestens eine Innenteil statt einer Längsdurchbrechung auch ein Sackloch aufweisen, in welchem ein das Innengewinde bildender Innengewindeabschnitt ausgebildet ist. Eine Längsdurchbrechung hat insbesondere den Vorteil, dass beispielsweise ein Gewindebolzen, welcher an einem Implantatteil angeordnet oder ausgebildet ist, oder auch ein Schraubbolzen beidseitig aus dem mindestens einen Innenteil herausragen können. Insbesondere kann so das mit der Mutter zu verschraubende Teil, welches ein Außengewinde aufweist, bei Bedarf gegengehalten werden, da ein Zugriff auf dieses möglich ist.

Günstig ist es, wenn das Innengewinde eine Innengewindelänge parallel zur Längsachse aufweist, wenn das mindestens eine Innenteil eine Innenteillänge parallel zur Längsachse aufweist und wenn die Innengewindelänge mindestens etwa der halben Innenteillänge entspricht. So kann eine besonders sichere und langzeitstabile Verschraubung realisiert werden. Insbesondere kann sich das Innengewinde auch über die gesamte Innenteillänge oder im Wesentlichen über die gesamte Innenteillänge erstrecken.

Um eine möglichst kompakte Bauform der medizinischen Mutter erreichen zu können, ist es günstig, wenn der Außenmehrkant eine Außenmehrkantlänge parallel zur Längsachse aufweist, wenn das mindestens eine Außenteil eine Außenteillänge parallel zur Längsachse aufweist und wenn die Außenmehrkantlänge höchstens etwa der halben Außenteillänge entspricht.

Vorzugsweise sind das mindestens eine Innenteil und das mindestens eine Außenteil in axialer Richtung parallel zur Längsachse unbeweglich oder im Wesentlichen unbeweglich miteinander gekoppelt. Auf diese Weise können Relativbewegungen des Innenteils und des Außenteils relativ zueinander parallel zur Längsachse minimiert oder vollständig eliminiert werden.

Günstig ist es, wenn das mindestens eine Innenteil einstückig oder einteilig ausgebildet ist und/oder wenn das mindestens eine Außenteil einstückig oder einteilig ausgebildet ist. Insbesondere kann so eine medizinische Mutter aus nur zwei Teilen ausgebildet werden, nämlich einem einzigen Innenteil und einem einzigen Außenteil.

Gemäß einer weiteren bevorzugten Ausführungsform der Erfindung kann vorgesehen sein, dass das mindestens eine Innenteil aus einem Innenteilmaterial ausgebildet ist, dass das mindestens eine Außenteil aus einem Außenteilmaterial ausgebildet ist und dass das Außenteilmaterial weicher und/oder elastischer ist als das Innenteilmaterial. Eine solche Ausgestaltung ermöglicht insbesondere eine sichere und definierte Verformbarkeit des mindestens einen Außenteils, um die Drehmomentbegrenzungsfunktion in der gewünschten Weise realisieren zu können. Insbesondere unterscheidet sich das Innenteilmaterial vom Außenteilmaterial. Eine elastischere Ausbildung des mindestens einen Außenteils kann insbesondere auch durch eine entsprechende Dimensionierung desselben erreicht werden, wenn das Innenteilmaterial und das Außenteilmaterial identisch sind.

Vorteilhafterweise ist ein Elastizitätsmodul des Innenteilmaterials größer als ein Elastizitätsmodul des Außenteilmaterials. So kann insbesondere sichergestellt werden, dass sich das mindestens eine Außenteil in gewünschter Weise verformen kann, um die Drehmomentbegrenzungsfunktion der Mutter zu erreichen.

Eine hinreichende Stabilität der medizinischen Mutter kann insbesondere dadurch erreicht werden, dass das Innenteilmaterial und das Außenteilmaterial Metalle oder metallische Werkstoffe sind oder enthalten. Insbesondere kann es sich um Legierungen von zwei, drei oder mehr Metallen handeln.

Günstig ist es, wenn das Innenteilmaterial ein Instrumentenstahl ist, welcher insbesondere Kobalt und/oder Chrom enthält, oder Nitinol oder Stahl. Insbesondere kann das Außenteilmaterial CoCrMo sein oder enthalten. Mit diesem Innenteilmaterial kann insbesondere ein hartes und stabiles Innenteil ausgebildet werden.

Vorteilhaft ist es ferner, wenn das Außenteilmaterial Titan enthält und/oder eine Titanlegierung ist, insbesondere Ti6Al4V. Die genannten Außenteilmaterialien sind insbesondere im Vergleich zu Titan oder Titan enthaltenden Innenteilmaterialien weicher und elastischer.

Günstig ist es, wenn die Mutter ein Außenteil in Form eines Klemmring umfasst, welcher distalseitig eine sich konisch verjüngende Außenfläche aufweist. Die Außenfläche kann insbesondere ein distales Ende der Mutter definieren. Mit der Außenfläche des Klemmrings kann die Mutter an einem der beiden miteinander zu verbindenden Implantatteile klemmend in Anlage gebracht werden. Der Klemmring ist vorzugsweise relativ zum Innenteil um die Längsachse verdrehbar angeordnet, parallel zur Längsachse jedoch unverschieblich oder im Wesentlichen unverschieblich.

Günstigerweise ist das Implantat in Form eines Gelenkimplantats oder eines Teils eines Gelenkimplantats ausgebildet. Derartige Implantate können insbesondere in Form von Revisionsimplantaten zum Einsatz werden. Revisionsimplantate werden in der Regel erst nach einer Explantation eines zuvor implantierten Implantats eingesetzt.

Die nachfolgende Beschreibung bevorzugter Ausführungsformen der Erfindung dient im Zusammenhang mit den Zeichnungen der näheren Erläuterung. Es zeigen:
- Figur 1:: eine perspektivische, teilweise durchbrochene Darstellung eines ersten Ausführungsbeispiels eines Implantats;
- Figur 2:: eine Schnittansicht längs Linie 2-2 in Figur 1;
- Figur 3:: eine perspektivische, teilweise geschnittene Darstellung einer medizinischen Mutter aus den Figuren 1 und 2;
- Figur 4:: eine Explosionsdarstellung der Anordnung aus Figur 3;
- Figur 5:: eine Schnittansicht längs Linie 5-5 in Figur 2;
- Figur 6:: eine Schnittansicht ähnlich Figur 5, jedoch mit einem wirkenden Anzugsdrehmoment, welches größer als ein Grenzdrehmoment ist;
- Figur 7:: eine Ansicht analog Figur 3 eines zweiten Ausführungsbeispiels einer medizinischen Mutter;
- Figur 8:: eine perspektivische, teilweise durchbrochene Explosionsdarstellung der Anordnung aus Figur 7;
- Figur 9:: eine Schnittansicht analog Figur 5 der Mutter aus Figur 7;
- Figur 10:: eine Schnittansicht analog Figur 6 der Mutter aus Figur 7;
- Figur 11:: eine Ansicht analog Figur 3 eines dritten Ausführungsbeispiels einer medizinischen Mutter;
- Figur 12:: eine Ansicht analog Figur 4 der Mutter aus Figur 11;
- Figur 13:: eine Schnittansicht analog Figur 5 der Mutter aus Figur 11;
- Figur 14:: eine Schnittansicht analog Figur 6 der Mutter aus Figur 11;
- Figur 15:: eine Ansicht analog Figur 3 eines vierten Ausführungsbeispiels einer medizinischen Mutter;
- Figur 16:: eine Ansicht analog Figur 4 der Mutter aus Figur 15;
- Figur 17:: eine Schnittansicht analog Figur 5 der Mutter aus Figur 15;
- Figur 18:: eine Schnittansicht analog Figur 6 der Mutter aus Figur 15
- Figur 19:: eine perspektivische Ansicht eines fünften Ausführungsbeispiels einer medizinischen Mutter;
- Figur 20:: eine perspektivische, teilweise geschnittene Ansicht der Mutter aus Figur 19;
- Figur 21:: eine ausschnittsweise Ansicht der Anordnung aus Figur 1 mit der Mutter aus Figur 19 in einer angezogenen Stellung;
- Figur 22:: eine Ansicht ähnlich Figur 21 mit der Mutter in einer nicht angezogenen Stellung;
- Figur 23:: eine Ansicht ähnlich Figur 2 mit einem weiteren Ausführungsbeispiel einer medizinischen Mutter und schematisch dargestelltem Ausdrehwerkzeug ;
- Figur 24:: eine Längsschnittansicht der Mutter aus Figur 23 mit schematisch dargestelltem Ausdrehwerkzeug mit selbstschneidendem Linksgewinde;
- Figur 25:: eine Längsschnittansicht des Außenteils der Mutter aus Figur 24;
- Figur 26:: eine Ansicht des Außenteils in Figur 25 in Richtung des Pfeils A;
- Figur 27:: eine Seitenansicht des Innenteils der Mutter aus Figur 24; und
- Figur 28:: eine Schnittansicht längs Linie 28-28 in Figur 27.

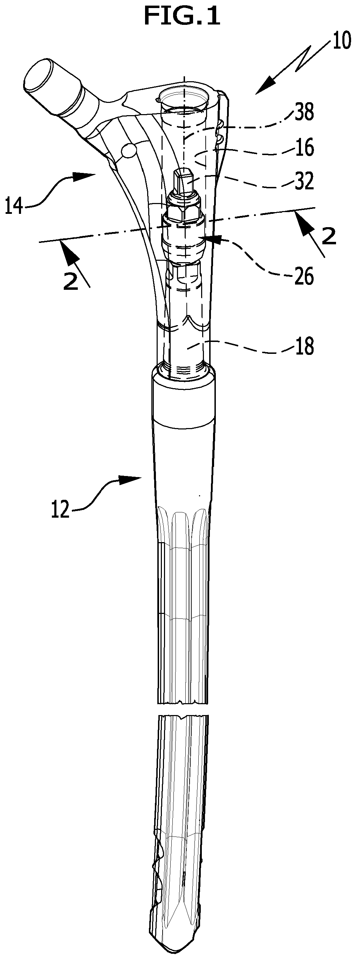

In Figur 1 ist beispielhaft ein mit dem Bezugszeichen 10 bezeichnetes Implantat in Form eines Revisionsschafts einer Hüftgelenkendoprothese zum Einsetzen in eine Kavität eines Femurs dargestellt.

Das Implantat 10 umfasst ein erstes Implantatteil 12 und ein zweites Implantatteil 14. Das erste Implantatteil 12 bildet einen proximalen Schaftabschnitt mit einer Längsdurchbrechung 16.

Das zweite Implantatteil 14 bildet eine Schaftverlängerung für das erste Implantatteil 12 und umfasst einen Kupplungsabschnitt 18, welcher in die Längsdurchbrechung 16 von distal her kommend eingesetzt ist.

Ein Innenquerschnitt der Längsdurchbrechung 16 verjüngt sich im Bereich einer Schulter 20 von proximal in distaler Richtung im Querschnitt.

Zum Verbinden der Implantatteile 12 und 14 ist am Kupplungsabschnitt 18 ein Außengewindeabschnitt 22 ausgebildet. Der Außengewindeabschnitt 22 schließt sich an ein proximales Ende 24 des Kupplungsabschnitts 18 an, welches als Außenvierkant ausgebildet ist.

Ferner ist eine medizinische Mutter 26 vorgesehen, die einen zum Außengewindeabschnitt 22 korrespondierenden Innengewindeabschnitt 28 aufweist. Die Mutter 26 umfasst ferner einen Außenmehrkant 30, der bei dem in den Figuren beispielhaft dargestellten ersten Ausführungsbeispiel der Mutter 26 in Form eines Außensechskant 32 ausgebildet ist.

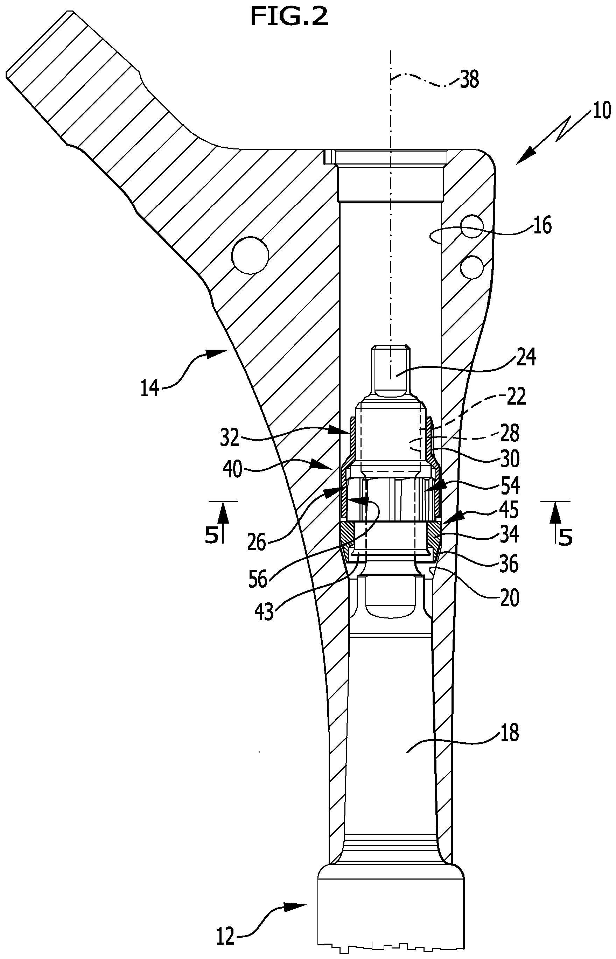

Wie in den Figuren 1 und 2 gut zu erkennen, ist das Ende 24 vollständig innerhalb der Längsdurchbrechung 16 angeordnet.

Distalseitig stützt sich die Mutter 26 an einem Klemmring 34 ab, welcher eine sich konisch verjüngende und an der Schulter 20 anliegende Außenfläche 36 aufweist.

Durch Anziehen der Mutter 26 im Uhrzeigersinn um eine Längsachse 38 derselben werden die Implantatteile 12 und 14 gegeneinander verspannt. Die Mutter 26 bildet in Verbindung mit dem Kupplungsabschnitt 18 eine Schraubverbindung 40 zum Verbinden der Implantatteile 12 und 14 in einer Verbindungsstellung, wie sie schematisch in den Figuren 1 und 2 dargestellt ist.

Das Implantat 10 kann insbesondere in Form eines Gelenkimplantats ausgebildet sein oder einen Teil eines solchen bilden, also beispielsweise einen schematisch in den Figuren 1 und 2 dargestellten Hüftschaft einer Hüftgelenkendoprothese.

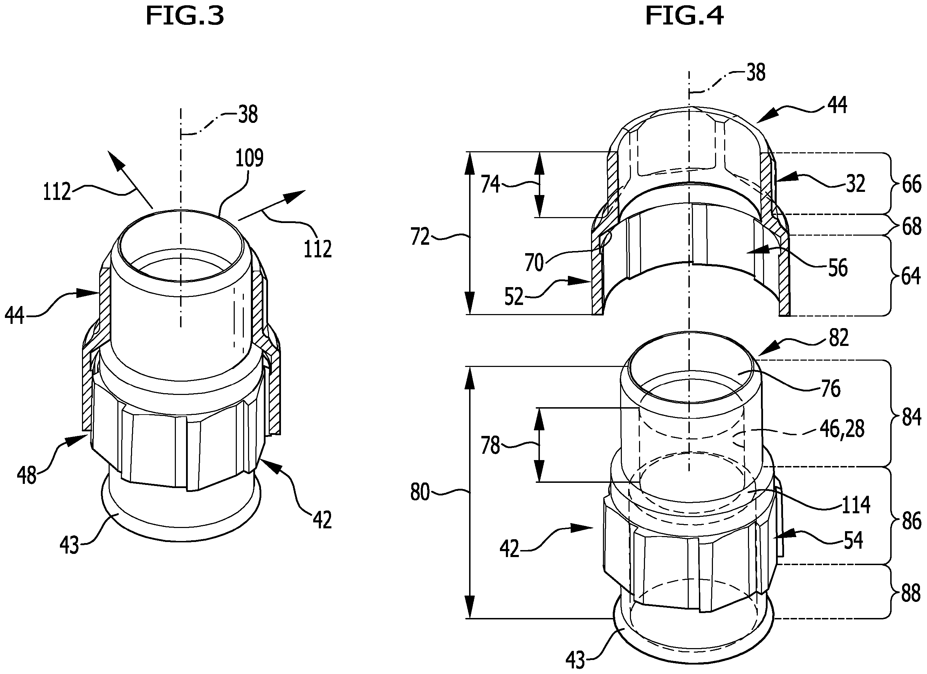

Der Aufbau und die Funktionsweise der Mutter 26 werden nachfolgend in Verbindung mit den Figuren 3 bis 6 näher erläutert.

Die Mutter 26 ist insgesamt zweiteilig ausgebildet und umfasst einen Innenteil 42 sowie ein das Innenteil 42 teilweise umgebendes Außenteil 44.

Das Innenteil 42 umfasst den ein Innengewinde 46 bildenden Innengewindeabschnitt 28. Das Außenteil 44 umfasst den Außenmehrkant 30.

Bei einem Ausführungsbeispiel ist der Klemmring 34 unlösbar mit der Mutter 26 gekoppelt, und zwar durch Verstemmen, also gezieltes plastisches Verformen von der Längsachse 38 weg, eines distalen Endes 43 des Innenteils 42. Durch die beschriebene unlösbare Kopplung umfasst die Mutter 26 insgesamt drei Komponenten, nämlich den Innenteil 42 sowie die Außenteile 44 und 45, wobei der Klemmring 34 und das Innenteil 42 relativ zueinander um die Längsachse 38 verdrehbar, jedoch axial im Wesentlichen unverschieblich aneinander gesichert sind.

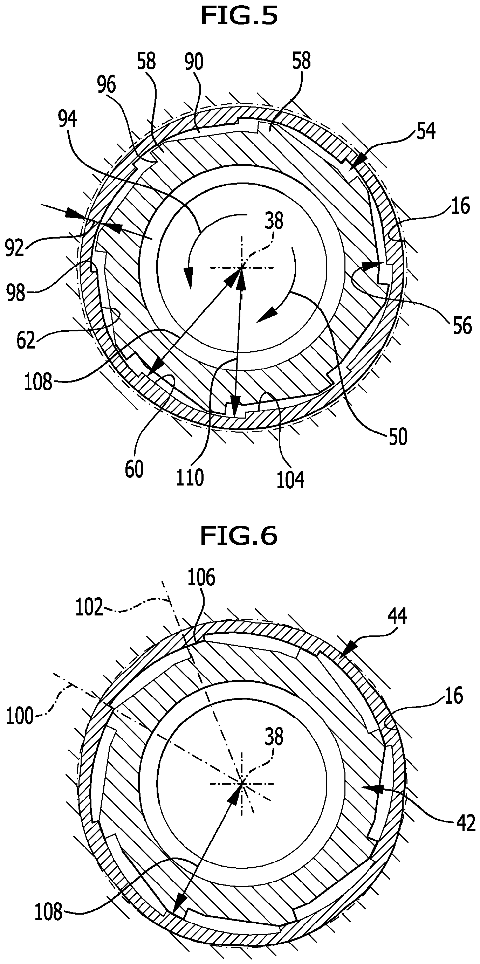

Die Mutter 26 umfasst ferner eine Drehmomentbegrenzungseinrichtung 48. Die Drehmomentbegrenzungseinrichtung 48 ist derart ausgebildet, dass bei einem in einer Anzugsdrehrichtung, symbolisiert in Figur 5 durch den Pfeil 50, auf das Außenteil 44 wirkenden Drehmoment, welches kleiner als ein Grenzdrehmoment ist, das Außenteilteil 44 und das Innenteil 42 in der Anzugsdrehrichtung relativ zueinander unverdrehbar sind. Ist dagegen das auf das Außenteil 44 in der Anzugsdrehrichtung wirkende Drehmoment gleich oder größer als das Grenzdrehmoment, so sind das Außenteil 44 und das Innenteil 42 in der Anzugsdrehrichtung relativ zueinander verdrehbar.

Das Grenzdrehmoment kann beispielsweise einen Wert in einem Bereich von etwa 2 Nm bis etwa 50 Nm aufweisen, insbesondere etwa 15 Nm.

Die Drehmomentbegrenzungseinrichtung 48 dient bei der in den Figuren 1 und 2 dargestellten Anordnung insbesondere dem Zweck, einen auf die zwischen der Schulter 20 und der an dieser anliegenden Außenfläche 36 des Klemmrings 34 ausgebildete Konusverbindung wirkenden Anpressdruck beim Anziehen der Mutter 26 zu begrenzen, um die Konusverbindung nicht zu beschädigen.

Die Funktion der Drehmomentbegrenzungseinrichtung 48 wird insbesondere dadurch erreicht, dass das Außenteil 44 verformbar ausgebildet ist und so eine temporäre Verformung zulässt. Das Außenteil 44, welches in Form einer Außenhülse 52 ausgebildet ist, lässt sich abhängig vom wirkenden Drehmoment verformen, und zwar derart, dass sich bei einem wirkenden Drehmoment, welches mindestens dem Grenzdrehmoment entspricht, das Außenteil 44 so verformt, dass es und das Innenteil 42 in der Anzugsdrehrichtung relativ zueinander verdrehbar sind.

Die Drehmomentbegrenzungseinrichtung 48 umfasst eine Innenteilverzahnung 54, die am Innenteil 42 angeordnet oder ausgebildet ist und von der Längsachse 38 in radialer Richtung weg weist.

Ferner umfasst die Drehmomentbegrenzungseinrichtung 48 eine Außenteilverzahnung 56, die am Außenteil 44 angeordnet oder ausgebildet ist. Die Außenteilverzahnung weist in radialer Richtung auf die Längsachse 38 hin und wirkt mit der Innenteilverzahnung 54 zusammen, und zwar derart, dass bei einem in der Anzugsdrehrichtung auf das Außenteil 44 wirkenden Drehmoment, welches kleiner als das Grenzdrehmoment der Drehmomentbegrenzungseinrichtung 48 ist, das Außenteil 44 und das Innenteil 42 in der Anzugsdrehrichtung 50 relativ zueinander unverdrehbar sind, jedoch bei einem in der Anzugsdrehrichtung auf das Außenteil 44 wirkenden Drehmoment, welches gleich oder größer als das Grenzdrehmoment ist, das Außenteil 44 und das Innenteil 42 in der Anzugsdrehrichtung relativ zueinander verdrehbar sind.

Die Innenteilverzahnung 54 umfasst mindestens einen Innenteilzahn 58. Bei dem in den Figuren 3 bis 6 dargestellten ersten Ausführungsbeispiel der Mutter 26 sind acht Innenteilzähne 58 ausgebildet, die in Umfangsrichtung gleichsinnig angeordnet und über einen Umfang des Innenteils 42 gleichmäßig verteilt angeordnet beziehungsweise ausgebildet sind.

Die Außenteilverzahnung 56 umfasst mindestens einen Außenteilzahn 60. Bei dem in den Figuren 3 bis 6 dargestellten ersten Ausführungsbeispiel der Mutter 26 sind insgesamt acht Außenteilzähne 60 auf einer Innenseite 62 der Außenhülse 52 angeordnet beziehungsweise ausgebildet und gleichmäßig über einen Umfang der Außenhülse 52 verteilt.

Das Außenteil 44 ist wie beschrieben hülsenförmig ausgebildet und umfasst einen distalen Hülsenabschnitt 64 und einen proximalen Hülsenabschnitt 66 auf. Ein Außendurchmesser des proximalen Hülsenabschnitts 66 ist kleiner als ein maximaler Außendurchmesser des distalen Hülsenabschnitts 64. Der proximale Hülsenabschnitt weist zudem einen kleineren Innendurchmesser auf als der distale Hülsenabschnitt 64.

Der distale Hülsenabschnitt 64 und der proximale Hülsenabschnitt 66 sind über einen Verbindungsabschnitt 68 miteinander verbunden welcher eine sich in distaler Richtung weisende, sich konisch erweiternde ringförmige Anschlagfläche 70 bildet.

Der distale Hülsenabschnitt 64 ist etwas länger als der proximale Hülsenabschnitt 66.

Das Außenteil 44 weist eine Außenteillänge 72 parallel zur Längsachse auf.

Der Außenmehrkant 30 ist ausschließlich am proximalen Hülsenabschnitt 66 ausgebildet. Er weist eine Außenmehrkantlänge 74 auf, die maximal der halben Außenteillänge 72 entspricht.

Die Außenteilverzahnung 56 ist ausschließlich im Bereich des distalen Hülsenabschnitts 64 ausgebildet. Die Außenteilzähne 60 erstrecken sich parallel zur Längsachse 38 somit in etwa auf einer Länge, die einer Länge des distalen Hülsenabschnitts 64 entspricht.

Das Innenteil 42 weist ferner eine sich koaxial zur Längsachse 38 erstreckende Längsdurchbrechung 76 auf. Das Innengewinde 46 ist im Bereich der Längsdurchbrechung 76 ausgebildet und umfasst den Innengewindeabschnitt 28.

Das Innengewinde 46 weist zudem eine Innengewindelänge 78 parallel zur Längsachse 38 auf. Eine Innenteillänge 80 parallel zur Längsachse 38 entspricht etwa einem dreifachen der Innengewindelänge 78.

Das Innenteil 42 ist in Form einer Innenhülse 82 ausgebildet, welche einen proximalen Hülsenabschnitt 84, einen mittleren Hülsenabschnitt 86 und einen distalen Hülsenabschnitt 88 definiert.

Der proximale Hülsenabschnitt 84 und der distale Hülsenabschnitt 88 werden auf ihrer Außenseite jeweils durch eine zylindrische Außenfläche begrenzt.

Der mittlere Hülsenabschnitt 86 ist mit der Innenteilverzahnung 54 versehen. Eine Länge des proximalen Hülsenabschnitts 84 und eine Länge des distalen Hülsenabschnitts 88 ist etwa gleich. Zudem sind der proximale Hülsenabschnitt 84 und der mittlere Hülsenabschnitt 86 jeweils etwa doppelt so lang wie der distale Hülsenabschnitt 88.

Das Außenteil 44 umfasst eine Außenteilwand 90, welche eine Dicke 92 aufweist, welche über einen Umfang des Außenteils 44 variiert. Mit anderen Worten ist die Außenteilwand 90 in Abhängigkeit eines Umfangswinkels unterschiedlich dick. Insbesondere weist die Dicke 92 der Außenteilwand 90 zwischen zwei Außenteilzähnen 60 ein Minimum auf.

Bei der Mutter 26 wirken ferner das Innenteil 42 und das Außenteil 44 in einer Löserichtung, in Figur 5 symbolisiert durch den Pfeil 94, derart zusammen, dass bei einer Verdrehung des Außenteils 44 um die Längsachse 38 in der der Anzugsdrehrichtung entgegengesetzt gerichteten Lösedrehrichtung das Innenteil 42 zusammen mit dem Außenteil 44 in der Lösedrehrichtung verdreht wird. Dies wird insbesondere dadurch erreicht, dass die Innenteilverzahnung 54 und die Außenteilverzahnung 56 bei Verdrehung des Außenteils 44 um die Längsachse 38 in der Lösedrehrichtung 94 ineinander greifen. Hierfür weist jeder Innenteilzahn 58 eine Innenteilzahnlöseflanke 96 auf und jeder Außenteilzahn 60 eine Außenteilzahnlöseflanke 98.

In einer Lösestellung liegen beim Verdrehen der Mutter 26 durch Einleiten eines Drehmoments auf das Außenteil 44 in der Löserichtung die Innteilzahnlöseflanke 96 und die Außenteilzahnlöseflanke 98 aneinander an. Der Innenteilzahn 58 bildet so einen Mitnehmer für das Außenteil 44 beziehungsweise jeder Außenteilzahn 60 bildet einen Mitnehmer für das Innenteil 42.

In Figur 6 ist beispielhaft eine Schnittebene 100 eingezeichnet, welche die Längsachse 38 enthält und parallel zur Außenteilzahnlöseflanke 98 verläuft.

Ferner ist in Figur 6 eine weitere, die Längsachse 38 enthaltende Schnittebene 102 eingezeichnet, die parallel zur Innenteilzahnlöseflanke 96 verläuft. Mithin schneiden die Innenteilzahnlöseflanke 96 und die Außenteilzahnlöseflanke 98 eine die Längsachse 38 enthaltende Schnittebene 100, 102 unter einem Winkel von 0°, verlaufen also jeweils parallel zu den Schnittebenen 100 beziehungsweise 102.

Jeder Außenteilzahn 60 weist eine Außenteilzahnaufgleitflanke 104 auf. Jeder Innenteilzahn 58 weist eine Innenteilzahnaufgleitflanke 106 auf.

Ein Abstand 108 einer in radialer Richtung nach außen weisenden, von der Innenteilzahnaufgleitflanke 106 definierten Seitenfläche von der Längsachse 38 ist etwas kleiner als ein Abstand 110 der Innenteilzahnaufgleitflanke 106 von der Längsachse 38. Dies hat zur Folge, dass bei einer Verdrehung des Außenteils 44 in der Anzugsdrehrichtung 50 relativ zum Innenteil 42 eine Verdrehung nur so lange ohne weiteres möglich ist, bis die Außenteilzahnaufgleitflanke 104 und die Innenteilzahnaufgleitflanke 106 aneinander aufgleiten und klemmend aneinander angreifen.

Aufgrund der unterschiedlichen Abstände 108 und 110 führt eine weitere Verdrehung in der Anzugsdrehrichtung dazu, dass bei einem wirkenden Drehmoment, das kleiner als das Grenzdrehmoment der Drehmomentbegrenzungseinrichtung 48 ist, die Außenteilzahnaufgleitflanke 104 und die Innenteilzahnaufgleitflanke 106 aneinander klemmend gehalten sind. Das Außenteil 44 und das Innenteil 42 können dann durch ein auf das Außenteil 44 in der Anzugsdrehrichtung wirkendes Drehmoment gemeinsam verdreht werden wie eine aus dem Stand der Technik bekannte, insgesamt einstückig ausgebildete Mutter.

Ist jedoch das auf das Außenteil 44 wirkende Drehmoment mindestens so groß wie das Grenzdrehmoment, so gleitet die Außenteilzahnaufgleitflanke 104 und die Innenteilzahnaufgleitflanke 106 weiter aneinander entlang. Aufgrund der besonderen Formgebung des Außenteils 44, welches durch die über den Umfang variierenden Dicke 92 zwischen zwei Außenteilzähnen 60 mindestens einen geschwächten und damit verformbaren Bereich umfasst, ist eine Verformung des Außenteils 44 wie schematisch in Figur 6 dargestellt derart möglich, dass das Außenteil 44 so aufgeweitet wird, dass der in einer Grundstellung, wie beispielhaft in Figur 5 dargestellt, eingezeichnete Abstand 108 so weit vergrößert wird, dass er im aufgeweiteten Zustand dem Abstand 110 entspricht.

Bei dem in den Figuren 3 bis 6 dargestellten ersten Ausführungsbeispiel der Mutter 26 sind die Außenteilzahnaufgleitflanken 104 flacher als die Außenteilzahnlöseflanken 98. Außerdem sind die Innenteilzahnaufgleitflanken 106 flacher als die Innenteilzahnlöseflanken 96. Flacher bedeutet hier insbesondere, dass ein Schnittwinkel von durch die jeweiligen Aufgleitflanken definierten Flächen mit einer die Radialebene schneidenden Schnittebene größer ist als ein Schnittwinkel dieser Schnittebene mit der jeweiligen Löseflanke.

Ferner sind bei den Figuren 3 bis 6 dargestellten Ausführungsbeispielen der Mutter 26 gleich viele Innenteilzähne 58 wie Außenteilzähne 60 ausgebildet.

Wie insbesondere in Figur 3 gut zu erkennen, steht das Innenteil 42 proximalseitig über das Außenteil 44 vor.

Des Weiteren sind das Innenteil 42 und das Außenteil 44 unlösbar miteinander gekoppelt. Dies wird dadurch erreicht, dass ein sich verjüngender Endabschnitt 109 des proximalen Hülsenabschnitts 84 in radialer Richtung, symbolisiert durch die Pfeile 112, nach außen verstemmt wird, so dass ein proximalseitiger Anschlag für das Außenteil 44 ausgebildet wird.

Das Außenteil 44 ist auf diese Weise zwischen dem Endabschnitt 109 und einem zwischen dem proximalen Hülsenabschnitt 84 und dem mittleren Hülsenabschnitt 86 ausgebildeten Übergangsbereich 114, welcher einen in distaler Richtung wirkenden Anschlag für das Außenteil 44 bildet, axial, das heißt parallel zur Längsachse 38, unbeweglich oder im Wesentlichen unbeweglich am Innenteil 42 gehalten.

Das Innenteil 42 ist ebenso wie das Außenteil 44 einstückig beziehungsweise einteilig ausgebildet. Auf diese Weise ist es möglich, die Mutter 26 lediglich aus zwei Teilen auszubilden, nämlich einem einzigen Innenteil 42 und einem einzigen Außenteil 44.

Um die beschriebene Eigenschaft des Außenteils 44, nämlich eine Verformung bei einem einwirkenden Drehmoment, welches gleich oder größer als das Grenzdrehmoment ist, zu erreichen, ist das Innenteil 42 vorzugsweise aus einem anderen Material ausgebildet als das Außenteil 44.

Ein Außenteilmaterial, aus dem das Außenteil 44 ausgebildet ist, ist vorzugsweise weicher und elastischer als ein Innenteilmaterial, aus dem das Innenteil 42 ausgebildet ist. Dies kann insbesondere dadurch erreicht werden, dass ein Elastizitätsmodul des Innenteilmaterials größer als ein Elastizitätsmodul des Außenteilmaterials.

Um eine hohe Stabilität der Mutter 26 zu erreichen, sind das Innenteilmaterial und das Außenteilmaterial vorzugsweise Metalle oder metallische Werkstoffe oder enthalten Metalle oder metallische Werkstoffe. Grundsätzlich ist es jedoch auch möglich, Kunststoffe und/oder Keramiken zur Ausbildung der Mutter 26 einzusetzen.

Ein hinreichend hartes und verformbares Innenteil 42 kann beispielsweise ausgebildet werden, wenn das Außenteilmaterial ein Instrumentenstahl ist, welcher insbesondere Kobalt und/oder Chrom enthält, oder Nitinol oder Stahl ist.

Die gewünschte Verformbarkeit bei Einwirkung hinreichend großer Drehmomente auf das Außenteil 44 kann insbesondere dadurch erreicht werden, dass das Innenteilmaterial Titan enthält und/oder eine Titanlegierung ist, beispielsweise Ti6Al4V.

Die beschriebene Mutter 26 ermöglicht es, dass das Außenteil 44 und das Innenteil 42 in der Löserichtung um die Längsachse 38 um einen Lösedrehwinkel von höchstens 360° relativ zueinander verdrehbar sind. Bei sowohl acht Innenteilzähnen 58 als auch acht Außenteilzähnen 60 beträgt somit ein maximaler Lösedrehwinkel bei dem in den Figuren 3 bis 6 dargestellten ersten Ausführungsbeispiel der Mutter 26 maximal 45°. Dieser Winkel wird insbesondere definiert durch einen Abstand der Innenteilzahnlöseflanke 96 voneinander beziehungsweise der Außenteilzahnlöseflanke 98 voneinander jeweils in Umfangsrichtung.

Außerdem sind das Außenteil 44 und das Innenteil 42 in der Anzugsdrehrichtung um die Längsachse 38 um einen Anzugsdrehwinkel von höchstens 360° relativ zueinander verdrehbar, wenn das Anzugsdrehmoment kleiner als das Grenzdrehmoment ist.

Bei dem in den Figuren 3 bis 6 dargestellten ersten Ausführungsbeispiel der Mutter 26 beträgt der Anzugsdrehwinkel maximal 45°. Dieser Winkel hängt vom Abstand benachbarter Außenteilzähne 60 beziehungsweise benachbarter Innenteilzähne 58 voneinander in Umfangsrichtung ab. Je kleiner die Anzahl der Zähne, umso größer der Anzugsdrehwinkel und umgekehrt.

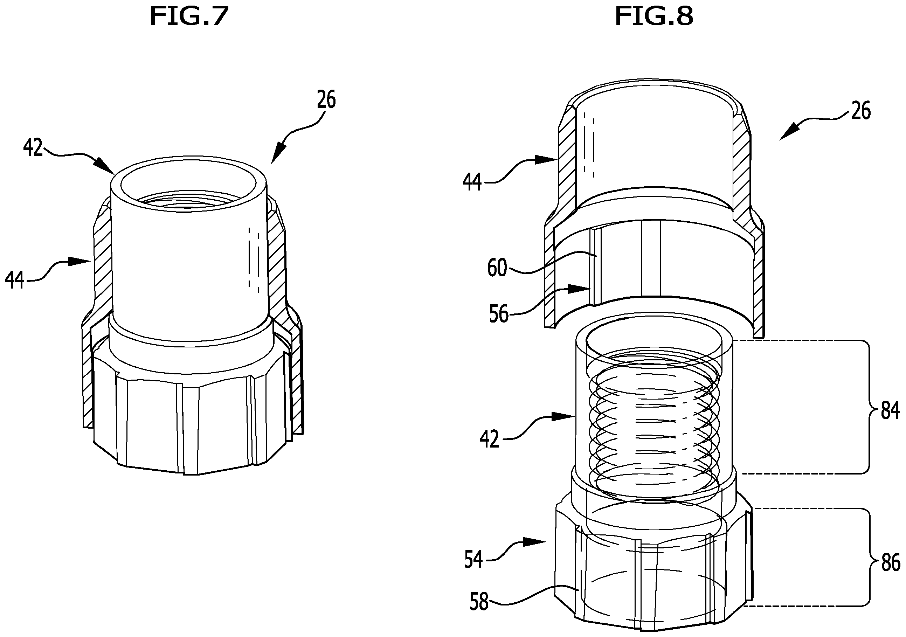

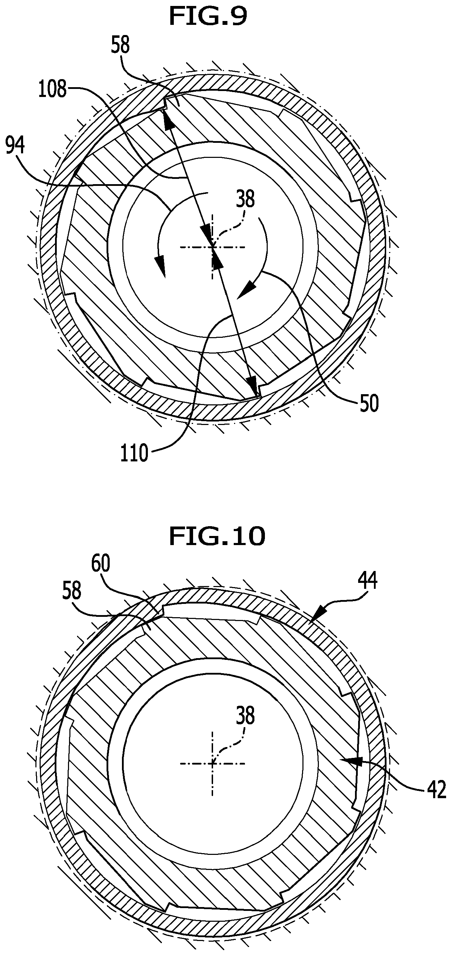

In den Figuren 7 bis 10 ist ein zweites Ausführungsbeispiel einer medizinischen Mutter 26 in vergleichbaren Ansichten wie in den Figuren 3 bis 6 dargestellt.

Der Unterschied zwischen dem zweiten Ausführungsbeispiel der Mutter 26 in den Figuren 7 bis 10 und dem ersten Ausführungsbeispiel der Mutter 26 in den Figuren 3 bis 6 besteht im Wesentlichen darin, dass die Innenteilverzahnung 54 lediglich einen einzigen Innenteilzahn 58 aufweist. Die Außenteilverzahnung 56 weist dagegen ebenfalls acht Außenteilzähne 60 auf.

Ein weiterer Unterschied besteht darin, dass beim zweiten Ausführungsbeispiel der Mutter 26 das Außenteil 44 distalseitig über das Innenteil 42 vorsteht. Dies wird insbesondere dadurch möglich, dass das Innenteil 42 des zweiten Ausführungsbeispiels keinen distalen Hülsenabschnitt 88 aufweist, sondern distalseitig durch den mittleren Hülsenabschnitt 86 begrenzt wird, welcher die Außenteilverzahnung 56 umfasst.

Auch beim zweiten Ausführungsbeispiel der Mutter 26 ermöglicht das Außenteil 44 eine Verformung derart, dass der Abstand 108 vergrößert wird, bis er dem Abstand 110 entspricht. So können das Innenteil 42 und das Außenteil 44 aneinander entlang gleiten, wenn das auf das Außenteil 44 wirkende Drehmoment gleich oder größer als das Grenzdrehmoment ist und in Anzugsdrehrichtung wirkt.

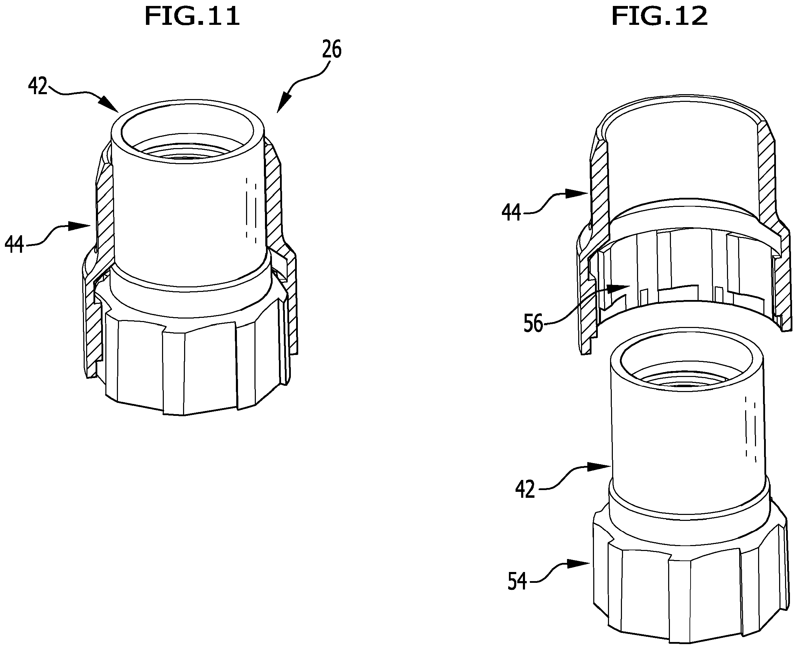

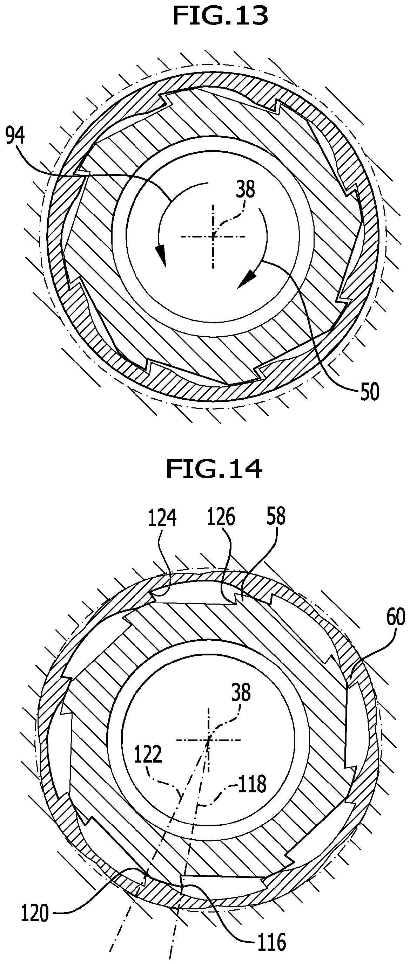

In den Figuren 11 bis 14 ist ein drittes Ausführungsbeispiel einer medizinischen Mutter beispielhaft dargestellt. Es entspricht in seinem Aufbau im Wesentlichen dem zweiten Ausführungsbeispiel, das in den Figuren 7 bis 11 dargestellt ist.

Das dritte Ausführungsbeispiel unterscheidet sich vom zweiten Ausführungsbeispiel insbesondere dadurch, dass das Innenteil 42 eine Innenteilverzahnung 54 mit acht Innenteilzähnen 58 aufweist.

Ferner sind sowohl die Innenteilzähne 58 als auch die Außenteilzähne 60 anders geformt. Beim dritten Ausführungsbeispiel sind sie hinterschnitten. Es ist also insbesondere jeweils die Innenteilzahnlöseflanke 96 bezogen auf eine die Längsachse 38 und eine Innenteilzahnspitze 116 des Innenteilzahns 58 enthaltende Radialebene 118 hinterschnitten. Ebenso ist auch die Außenteilzahnlöseflanke 96 bezogen auf eine die Längsachse 38 und eine Außenteilzahnspitze 120 des Außenteilzahns 60 enthaltene Radialebene 122 hinterschnitten.

Werden das Innenteil 42 und das Außenteil 44 in der Löserichtung relativ zueinander verdreht, greifen die Innenteilzähne 58 und die Außenteilzähne 60 jeweils in Hinterschneidungen 124 beziehungsweise 126 des jeweils anderen Zahns ein. So kann das Lösen der Mutter 26 besonders sicher erreicht werden.

Auch beim dritten Ausführungsbeispiel der Mutter 26 wird eine Drehmomentbegrenzungsfunktion durch eine definierte Verformbarkeit des Außenteils 44 erreicht in Folge eines auf das Außenteil 44 einwirkenden Drehmoments bei Verdrehung des Innenteils 42 und des Außenteils 44 relativ zueinander in der Anzugsdrehrichtung.

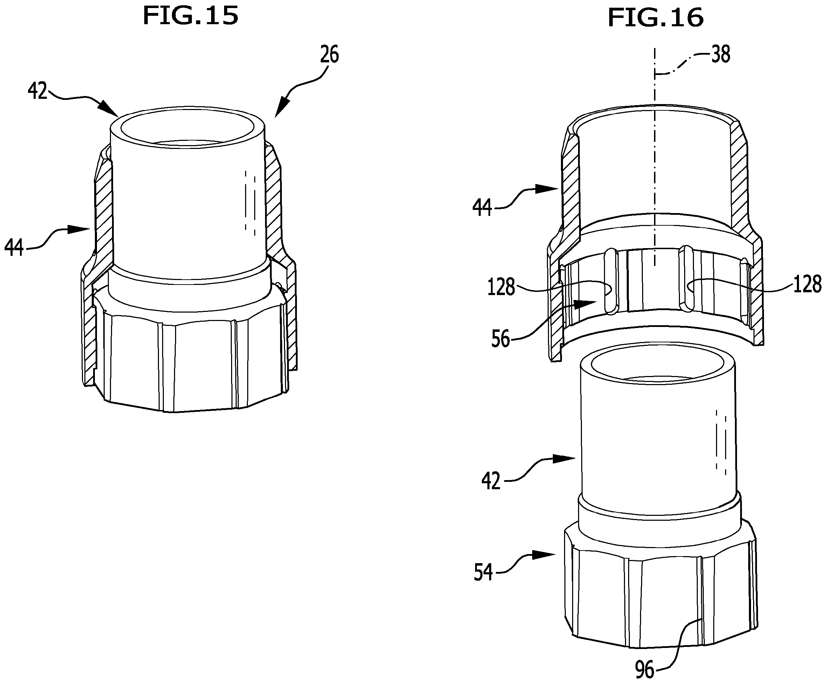

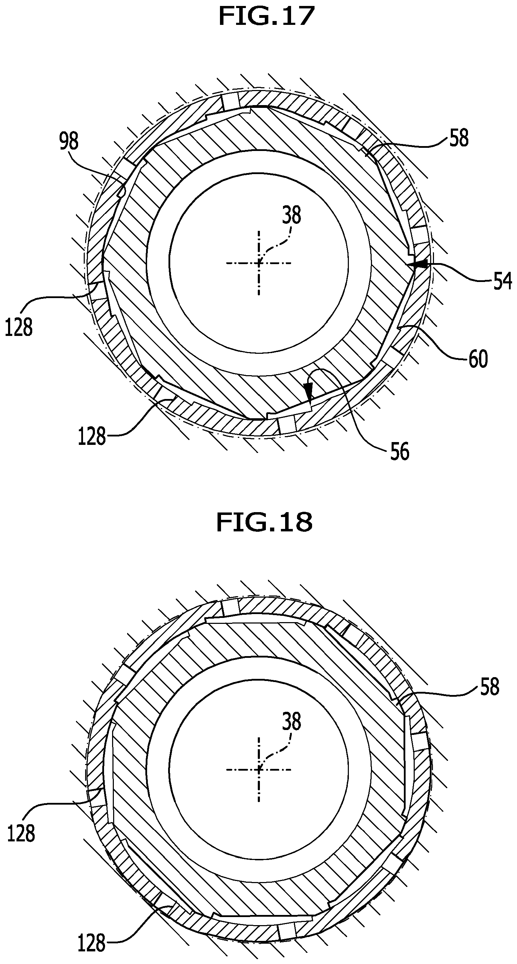

In den Figuren 15 bis 18 ist ein viertes Ausführungsbeispiel einer medizinischen Mutter 26 schematisch dargestellt. Es ist in seinem Aufbau dem dritten Ausführungsbeispiel ähnlich. Es umfasst ebenfalls acht Innenteilzähne 58 und acht Außenteilzähne 60 zur Ausbildung der Innenteilverzahnung 54 und der Außenteilverzahnung 56. Allerdings sind beim vierten Ausführungsbeispiel die Innenteilzahnlöseflanken 96 in axialer Richtung etwas in Bezug zur Längsachse 38 geneigt.

Des Weiteren sind am Außenteil 44 entsprechend der Anzahl der Außenteilzähne 60 acht Schlitze 128 ausgebildet und gleichmäßig über den Umfang verteilt. Sie sind in Umfangsrichtung etwas beabstandet von den Außenteilzahnlöseflanken 98 angeordnet und weisen eine Länge parallel zur Längsachse 38 auf, die etwa dreimal bis viermal so groß ist wie eine Breite der Schlitze 128 in Umfangsrichtung.

Durch die Schlitze 128 wird das Außenteil 44 definiert geschwächt, so dass bei einem auf das Außenteil 44 in Anzugsdrehrichtung wirkenden Drehmoment eine definierte Verformung des Außenteils 44 möglich ist, wenn das Drehmoment das Grenzdrehmoment erreicht oder überschreitet.

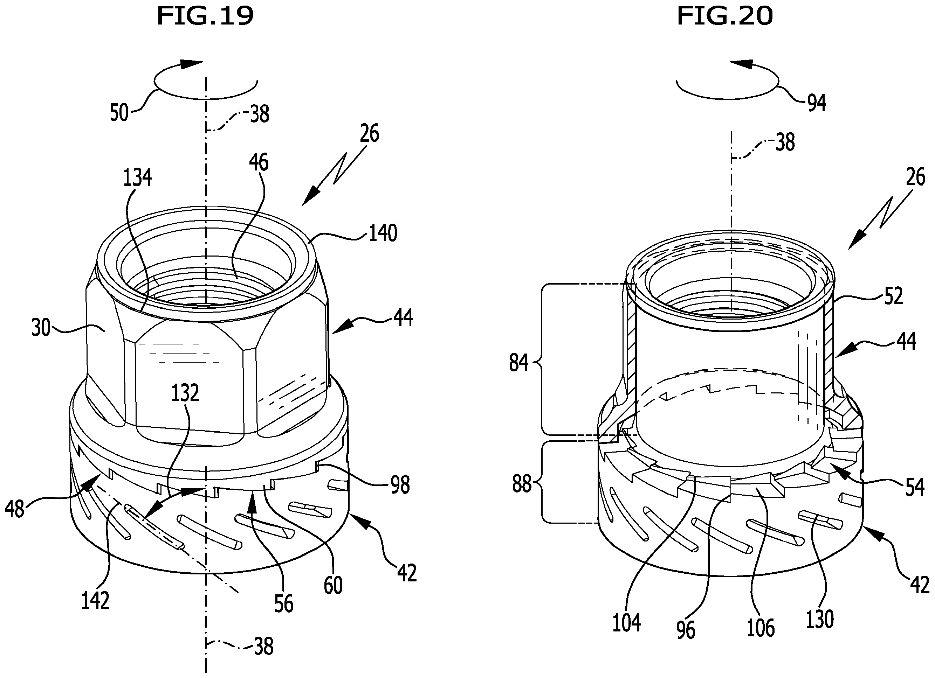

In den Figuren 19 bis 22 ist ein fünftes Ausführungsbeispiel einer medizinischen Mutter 26 schematisch dargestellt. Es unterscheidet sich in seinem Aufbau von den Ausführungsbeispielen 1 bis 4 der Mutter 26 in mehreren Aspekten.

Die Mutter 26 gemäß dem fünften Ausführungsbeispiel umfasst ein Außenteil 44 in Form einer Außenhülse 52. Diese ist mit einem Außenmehrkant 30 in Form eines Außensechskants 32 versehen.

Proximalseitig definiert das Außenteil 44 eine in proximaler Richtung weisende ringförmige Randfläche 134. Distalseitig wird das Außenteil 44 begrenzt durch eine in distaler Richtung, also in axialer Richtung, weisende Außenteilverzahnung 46 mit einer Mehrzahl von Außenteilzähnen 60, die einen in distaler Richtung weisenden Rand definieren.

Die Außenteilzähne 60 weisen jeweils eine Außenteilzahnlöseflanke 98 auf, die parallel zu einer die Längsachse 38 enthaltenden Ebene verläuft, sowie eine Außenteilzahnaufgleitflanke 104, die bezogen auf eine senkrecht zur Längsachse 38 verlaufende Ebene 136 um etwa 10° geneigt ist.

Die Mutter 26 umfasst ferner ein Innenteil 42, welches hülsenförmig ausgebildet ist und einen proximalen Hülsenabschnitt 84 sowie einen sich an diesen anschließenden, jedoch etwas größeren Außendurchmesser aufweisenden distalen Hülsenabschnitt 88 aufweist.

Im Bereich des proximalen Hülsenabschnitts 84 ist am Innenteil 42 ein Innengewinde 46 ausgebildet.

Am distalen Hülsenabschnitt 88 ist in proximaler Richtung weisend eine Innenteilverzahnung 54 ausgebildet, die eine Mehrzahl von Innenteilzähnen 58 aufweist, die in proximaler Richtung, also in axialer Richtung, weisen.

Jeder Innenteilzahn 58 weist eine Innenteilzahnlöseflanke 96 und eine Innenteilzahnaufgleitflanke 106 auf. Jede Innenteilzahnlöseflanke 96 verläuft parallel zu einer die Längsachse 38 enthaltenden Ebene. Die Innenteilzahnaufgleitflanke 106 ist ebenso wie die Außenteilzahnaufgleitflanke 104 relativ zur Ebene 136 um einen Winkel 138 um etwa 10° geneigt.

Der proximale Hülsenabschnitt 84 durchsetzt das hülsenförmige Außenteil 44. Ferner ist am proximalen Ende des Innenteils 42 ein in radialer Richtung von der Längsachse 38 weg weisend abstehender ringförmiger Flansch 140 ausgebildet. Der Flansch 140 bildet einen Anschlag für das Außenteil 44, so dass dieses mit der Randfläche 134 maximal so weit in proximaler Richtung bewegt werden kann, bis die Randfläche 134 am Flansch 140 anschlägt.

Distalseitig bildet Innenteilverzahnung 54, die in proximaler Richtung weist, einen Anschlag für die Außenteilverzahnung 56, so dass das Außenteil nicht weiter in distaler Richtung bewegt werden kann.

Im Bereich des distalen Hülsenabschnitts 88 ist eine Mehrzahl von Schlitzen 130 ausgebildet, die in Form langlochartiger Durchbrechungen ausgebildet sind. Längsachsen 142 der Schlitze 130 sind bezogen auf die Längsachse 38 um einen Winkel 132, welcher etwa 30° beträgt, geneigt.

Die Funktionsweise des fünften Ausführungsbeispiels der Mutter 26 wird nachfolgend erläutert.

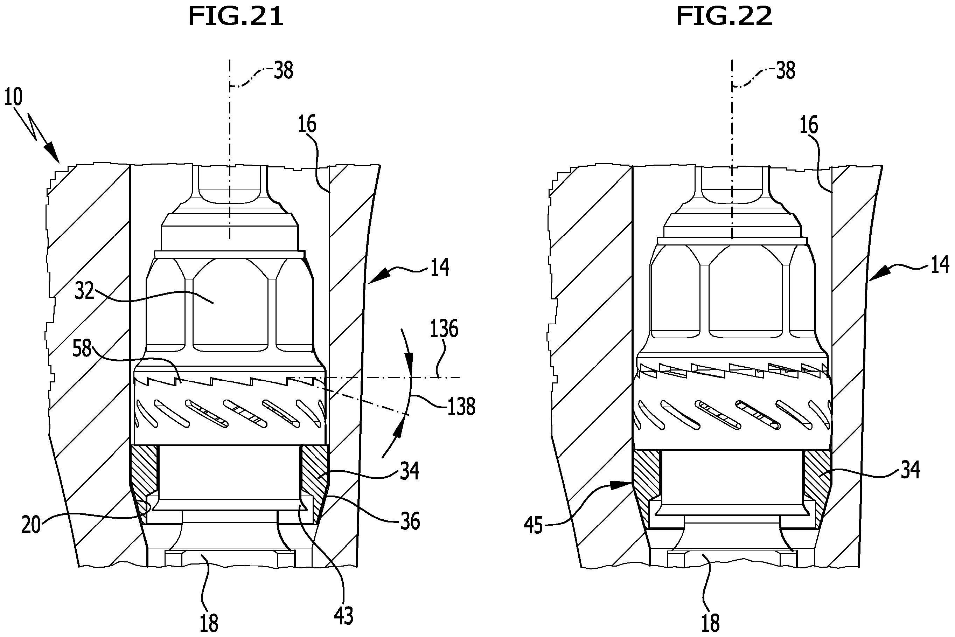

Wirkt auf das Außenteil 44 ein Drehmoment in Richtung des Pfeils 50, welcher eine Anzugsdrehrichtung definiert, so gleiten die Außenteilzahnaufgleitflanken 104 ausgehend von einer Grundstellung, wie sie beispielhaft in Figur 21 dargestellt ist, auf den Innenteilzahnaufgleitflanken 106 so lange auf, bis das für die Mutter 26 definierte Grenzdrehmoment erreicht ist. Figur 22 zeigt diese Situation kurz vor Erreichen des Grenzdrehmoments. Wird dieses überschritten, erlauben die Schlitze 130 eine Verformung des Innenteils 42, und zwar im Bereich des distalen Hülsenabschnitts 88, so dass das Außenteil 44 das Innenteil 42 nicht mehr mitnehmen kann. Die Innenteilverzahnung 54 und die Außenteilverzahnung 56 verdrehen sich dabei um eine Zahnlänge in Umfangsrichtung relativ zueinander, so dass die Innenteilzahnlöseflanken 96 und die Außenteilzahnlöseflanken 98 einen Zahn weiter einander hintergreifen können. Sobald dieses Hintergreifen eintritt, kann sich der distale Hülsenabschnitt 88 etwas in seine Grund- oder Ausgangsstellung zurückverformen. Dies erfolgt ruckartig und kann so vom Operateur, der die Mutter 26 anzieht, sowohl haptisch als auch akustisch wahrgenommen werden.

Sobald dieses Hinterschnappen der Innenteilzähne 58 und der Außenteilzähne 60 vom Operateur gespürt oder gehört wird, weiß er, dass das maximale Anzugsdrehmoment, das durch die konkrete Konstruktion der Mutter 26 mit ihrer Drehmomentbegrenzungseinrichtung 48 vorgegeben wird, überschritten ist. Der Operateur muss in diesem Fall die Mutter 26 nicht weiter anziehen. Dies ist aufgrund der besonderen Konstruktion auch nur eingeschränkt möglich, da sich beim Anziehen der Mutter 26 der distale Hülsenabschnitt 88 etwas verkürzt. Eine solche Verkürzung ist jedoch nur begrenzt möglich, und zwar nur so wei, wie dies die Schlitze 130 aufgrund ihrer Abmessungen und ihrer Orientierung zulassen.

Um die Mutter 26 gemäß dem fünften Ausführungsbeispiel wieder zu lösen, wird in der durch den Pfeil 94 symbolisierten Drehrichtung ein Lösedrehmoment auf das Außenteil 44 eingeleitet. Das Innenteil 42 und das Außenteil 44 können relativ zueinander nur soweit verdreht werden, bis die Innenteilzahnlöseflanken 96 und die Außenteilzahnlöseflanken 98, wie beispielhaft in Figur 21 dargestellt, aneinander anliegen. Die Innenteilzähne 58 und die Außenteilzähne 60 dienen dann gegenseitig als Mitnehmer, so dass eine Verdrehung des Außenteils 44 zu einer entsprechenden Verdrehung des Innenteils 42 führt.

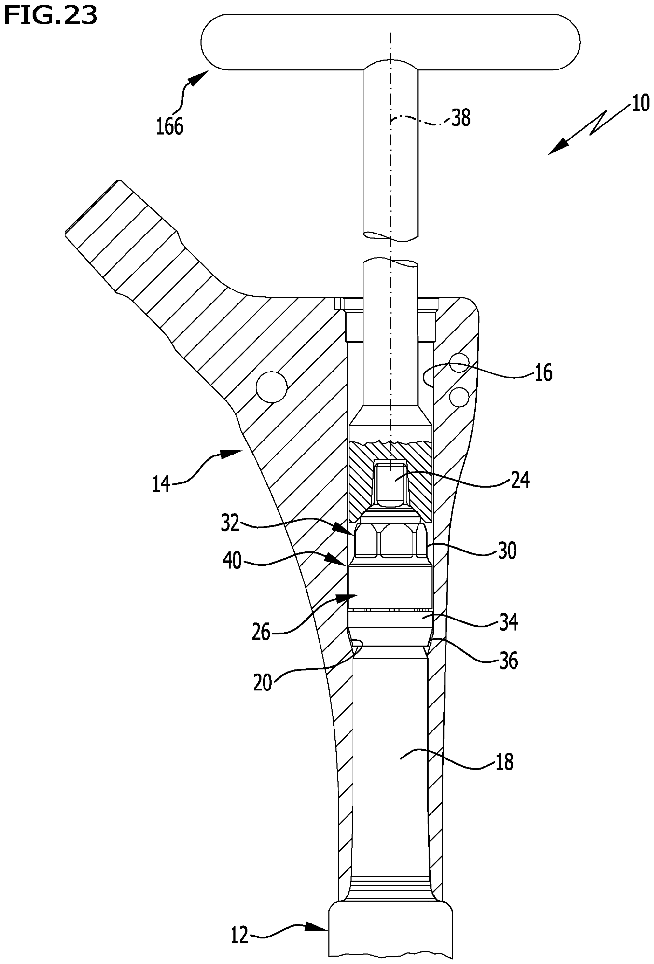

Ein weiteres Ausführungsbeispiel eines Implantats 10 ist schematisch in Figur 23 dargestellt. Auch bei diesem wird ein erstes Implantatteil 12 mit einem zweiten Implantatteil 14 verschraubt. Das zweite Implantatteil 14 bildet ebenfalls eine Schaftverlängerung für das erste Implantatteil 12.

Die Implantatteile 12 und 14 entsprechen in ihrem Aufbau im Wesentlichen den Implantatteilen 12 und 14, wie sie in Verbindung mit dem ersten Ausführungsbeispiel eines Implantats im Zusammenhang mit den Figuren 1 und 2 erläutert wurden.

Zum Koppeln der Implantatteile 12 und 14 dient wiederum eine Schraubverbindung 40. Eine Mutter 26 wird mit ihrem Innengewindeabschnitte 28 auf den Außengewindeabschnitt 22 des Kupplungsabschnitts 18 des zweiten Implantatteils 14 aufgeschraubt.

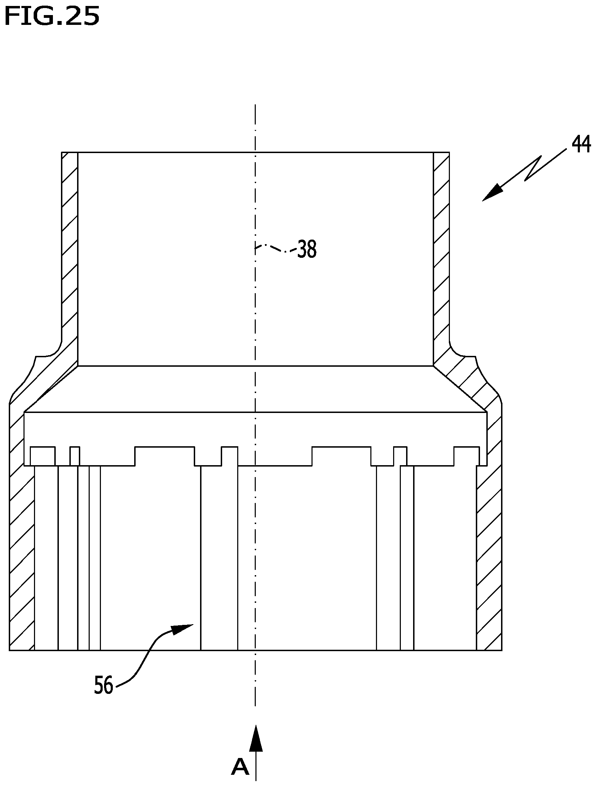

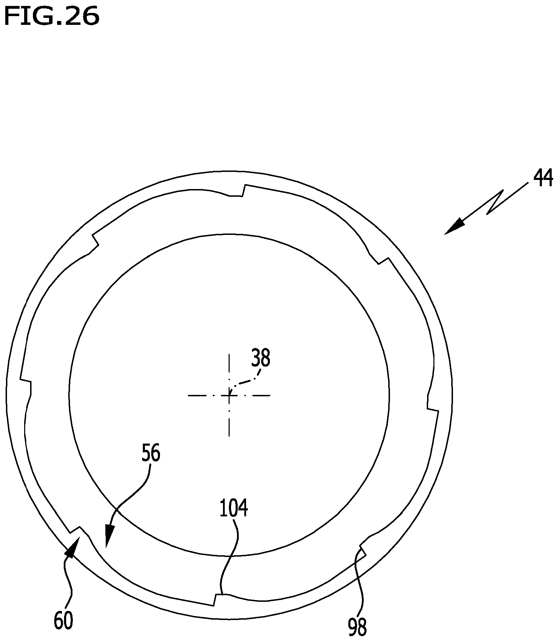

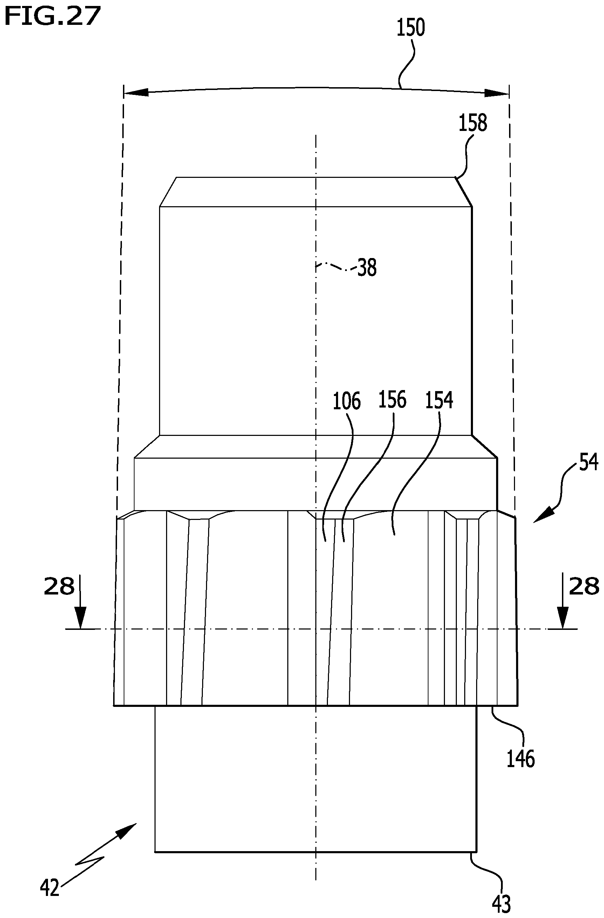

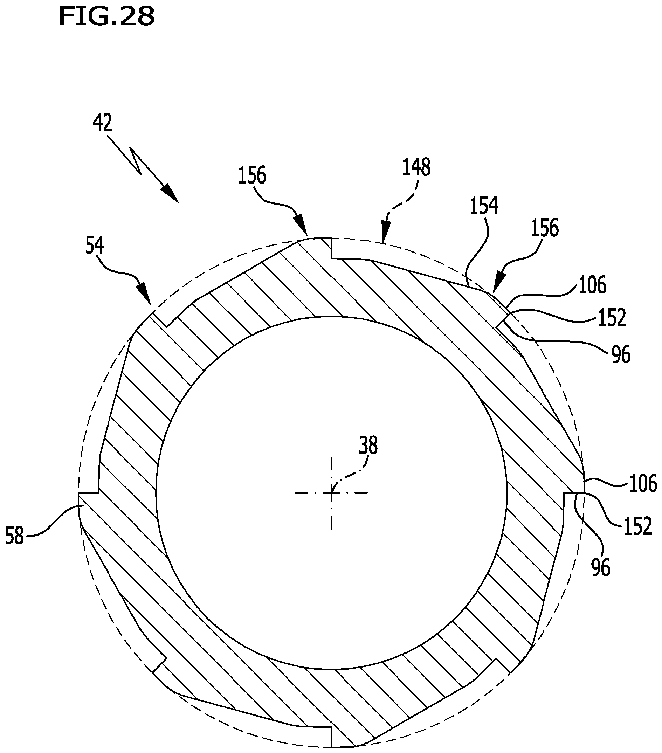

Die Mutter 26 bei dem in den Figuren 23 bis 28 dargestellten Ausführungsbeispiel stimmt in ihrem Aufbau im Wesentlichen mit der Mutter 26 des in den Figuren 1 bis 6 dargestellten Ausführungsbeispiels überein.

Auf dem distalen Hülsenabschnitt 88 ist wiederum ein Klemmring 34 gelagert, welcher mit einem in distaler Richtung vorspringenden Ende 35 ein distales Ende 35 der Mutter 26 bildet.

Eine in proximaler Richtung weisende Endfläche 144 des Klemmrings 34 liegt an einer in distaler Richtung weisenden Ringfläche 146 des Innenteils 42 an.

Ein distales Ende 43 des Innenteils 42 ist verstemmt, das heißt etwas von der Längsachse 38 weg nach außen verformt, so dass der Klemmring 34 zwar um die Längsachse 38 verdrehbar, jedoch in axialer Richtung am Innenteil 42 unverlierbar gesichert ist. Der Klemmring 34 kann nur entfernt werden, wenn die plastische Verformung des Innenteils 42 im Bereich des Endes 43 gewaltsam rückgängig gemacht wird. Dies führt jedoch zu einer Beschädigung der Mutter 26 und macht sie unbrauchbar.

Die Mutter 26 des in den Figuren 23 bis 28 dargestellten Ausführungsbeispiels stimmt in ihrem Aufbau wie erwähnt im Wesentlichen mit der in den Figuren 1 bis 6 dargestellten Mutter 26 überein. Allerdings gibt es auch Unterschiede.

So ist insbesondere die Innenteilverzahnung 54, die eine Außenkontur 148 definiert, in Richtung auf das Ende 35 hin sich konisch erweiternd ausgebildet. Die Außenkontor 148, die auch als einhüllende Konusfläche der Innenteilverzahnung 54 definiert werden kann, weist einen Konuswinkel 150 auf, der in einem Bereich von etwa 0 Grad bis 5 Grad liegt, bevorzugt etwa 1,5 Grad beträgt.

Die Innenteilverzahnung 54 ist ferner derart ausgebildet, dass die Innteilzahnaufgleitflanken 106 und die Innenteilzahnlöseflanken 96 scharfe Kanten 152 ausbilden.

An die Innenteilzahnaufgleitflanken 106 schließt sich jeweils eine Innenteilzahnaußenfläche 154 an. Die Innenteilzahnaufgleitflanke 106 und die Innenteilzahnaußenfläche 154 sind etwas gegeneinander geneigt. In einem Übergangsbereich 156 geht die Innenteilzahnaufgleitflanke 106 in die Innenteilzahnaußenfläche 154 über. Dieser Übergangsbereich 156 ist jedoch nicht scharfkantig wie die Kante 152, sondern verrundet. Ein Radius des Übergangsbereichs liegt bei dem beschriebenen Ausführungsbeispiel in einem Bereich von etwa 0,5 mm bis etwa 3 mm und beträgt insbesondere 1,5 mm.

Das Innenteil 42 umfasst acht die Innenteilverzahnung 54 bildende Innenteilzähne 58. Das Außenteil 44 umfasst acht die Außenteilverzahnung 56 bildende Außenteilzähne 60.

Mithin ist bei dem beschriebenen Ausführungsbeispiel der Mutter 26 ein Zahnverhältnis, das definiert wird durch ein Verhältnis der Anzahl der Außenteilzähne 60 und der Anzahl der Innenteilzähne 58, eins. Somit liegt dieses Zahnverhältnis also in einem Bereich von etwa 0,7 bis etwa 1,3.

Der Klemmring 34 ist unverzahnt ausgebildet. Insbesondere umfasst er keine Innenteilverzahnung wie das Innenteil 42.

Um das Außenteil 44 unverlierbar am Innenteil 42 zu sichern, ist ein proximales Ende 158, ähnlich wie das Ende 43, durch plastische Verformung in einer Richtung von der Längsachse 38 weg verstemmt. So kann das Innenteil 42 nicht parallel zur Längsachse 38 relativ zum Außenteil in distaler Richtung verschoben werden. Eine Rotation des Innenteils 42 und des Außenteils 44 relativ zueinander um die Längsachse 38 ist in der beschriebenen Weise jedoch möglich.

Durch das Verstemmen der Enden 43 und 158 am Innenteil 42 sind die drei Teile der Mutter 26 in bestimmungsgemäßer Weise unlösbar miteinander gekoppelt und unverlierbar aneinander gesichert. Eine Drehmomentbegrenzungsfunktion wird wie bei den anderen oben beschriebenen Ausführungsbeispielen durch das Zusammenwirken zwischen dem Innenteil 42 und dem Außenteil 44 erreicht.

Das Außenteil 44 ist bei diesem Ausführungsbeispiel geschlossen ausgebildet, weist also keine Schlitze auf.

Die besondere Ausgestaltung der Mutter 26 ermöglicht es, trotz der räumlichen Enge im Bereich des zweiten Implantatteils, die sich durch Abmessungen der Durchbrechung 16 ergibt, welche einen maximalen Durchmesser von 13,8 mm aufweist, die Schraubverbindung 40 zu realisieren. Durch die räumliche Vorgabe beim Implantat 10 weisen die Innteilverzahnung 54 und die Außenteilverzahnung 56, die miteinander zusammenwirken zur Drehmomentbegrenzung eines Anzugsdrehmoments, eine Höhe 160 von maximal etwa 7 mm auf. Unter Berücksichtigung dieser Abmessungen steht rein rechnerisch nur ein Hebel von etwa 6 mm zur Verfügung, mit welchem jedoch ein Anzugsdrehmoment von etwa 15 Nm übertragen werden muss.

Ferner beträgt eine Gesamthöhe 162 der Mutter zwischen dem Ende 35 und dem Ende 158 maximal 24 mm.

Die Innenteilverzahnung 54 mit der konischen Außenkontur 148 ist vorgesehen, damit sich die Innenteilzähne 58 auch noch nach einer Aufweitung des Außenteils 44 infolge des Überschreitens des vorgegebenen Anzugsdrehmoments optimal an die Außenteilzähne 60 anpassen und mit diesen zusammenwirken können. Die Außenteilzahnaufgleitflanken 104 und die Innteilzahnaufgleitflanken 106 liegen auf diese Weise stets gleichmäßig und vorzugsweise mindestens linienförmig, vorzugsweise flächig, aneinander an, so dass keine punktuelle Kraftübertragung auftreten kann.

Der Radius im Übergangsbereich 156 verhindert beim Verdrehen des Innenteils 42 und des Außenteils 44 relativ zueinander ein Blockieren in der Anzugsdrehrichtung.

Je mehr Innenteilzähne 58 und Außenteilzähne 60 beim Einsatz der Mutter 26 in Eingriff stehen, umso besser lassen sich Anzugsdrehmomente übertragen. Das eingeleitete Anzugsdrehmoment verteilt sich dann auf eine größere Fläche. Ein Verschleiß wird reduziert und eine Langlebigkeit der Mutter 26 wird erhöht.

Durch das Verstemmen der Enden 43 und 158 des Innenteils 42 können die Komponenten der Mutter 26 nicht voneinander getrennt werden, insbesondere auch nicht beim Einsatz der Mutter 26 in einer Klinik. So kann verhindert werden, dass einzelne Teile der Mutter 26 verloren gehen können, insbesondere im Körper eines Patienten bei der Implantation des Implantats 10.

Das Außenteil 44 ist aus einer Titanlegierung ausgebildet, beispielsweise Ti6Al4V. Alternativ kann es bei Ausführungsbeispielen auch aus einem Stahl ausgebildet sein. Beide genannten Materialien weisen praktisch keine elastische Dehnung in radialer Richtung auf, wenn sie durch Verdrehen des Innenteils 42 und des Außenteils 44 relativ zueinander von innen mit Druck beaufschlagt werden. Vielmehr kommt es zu einer im Wesentlichen plastischen Verformung des Außenteils 44.

Nach dem Zusammenfügen des Innenteils 42, des Außenteils 44 und des Klemmrings 34 bei der Montage der Mutter 26 wird diese durch mehrmaliges Betätigen kalibriert beziehungsweise eingestellt. Dabei verformt sich das Außenteil 44 etwas radial und weitet sich in Richtung auf sein distales Ende 164 konisch auf. Nach den sogenannten ersten gut hörbaren Klicks, die durch das Hinterschnappen der Innenteilzähne 58 und der Außenteilzähne 60 beim Überschreiten des maximalen Anzugsdrehmoments erzeugt werden, liegt dieses zunächst deutlich über den gewünschten 15 Nm, nämlich bei über 20 Nm. Durch wiederholtes Hinterschnappen der Innenteilzähne 58 und der Außenteilzähne 60 beim Betätigen der Mutter 26 in Anzugsdrehrichtung pendelt sich das Grenzdrehmoment bei etwa 15 Nm ein. Das Außenteil 44 ist dann wie beschrieben etwas konisch aufgeweitet und dauerhaft plastisch verformt. Eine Überlappung der Innenteilzähne 58 und der Außenteilzähne 60 ist dann sehr gering und liegt bei unter 0,05 mm.

Die wie beschrieben kalibrierte beziehungsweise eingestellte Mutter 26 kann nun zum Verbinden der Implantatteile 12 und 14 eingesetzt werden.

Die Mutter 26 wird bei einem Ausführungsbeispiel als Einmalteil angeboten. Vorzugsweise ist sie steril verpackt.

Die Mutter 26 ist für etwa 10 bis 15 Überdrehungen ausgebildet, die durch die beschriebenen Klicks hörbar sind. Eine mehrmalige Verwendung der Mutter 26 kann durch Verschleiß zum Versagen führen.

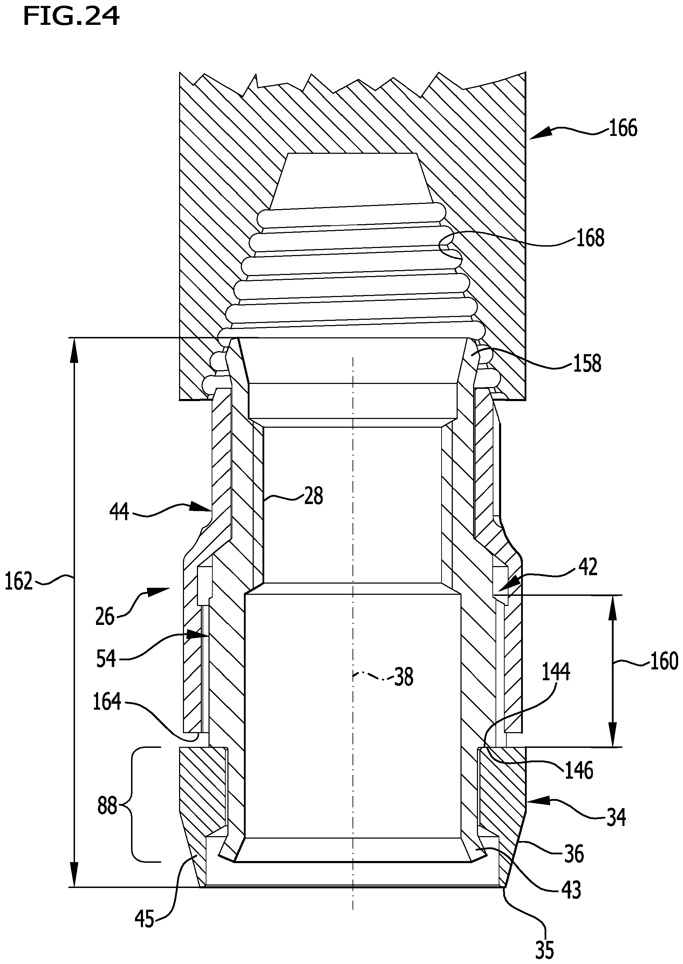

Um die Mutter 26 im Versagensfall wieder vom Außengewindeabschnitt 22 des Kupplungsabschnitts 18 des ersten Implantatteils 12 lösen zu können, dient ein in den Figuren 23 und 24 schematisch dargestellte Ausdrehwerkzeug 166. Dieses weist ein ausgehend von einem hülsenförmigen distalen Ende ein konisches Innengewinde 168 auf, welches sich in proximaler Richtung verjüngt.

Damit das Innengewinde 168, welches vorzugsweise selbstschneidend ausgebildet ist, mit der Mutter 26 zusammenwirken kann, steht das Ende 158 etwas über den Außensechskant 32 in proximaler Richtung vor. Dies ermöglicht es im beschriebenen Notfall, das Ausdrehwerkzeug 166 mit seinem distalen Ende 158 auf die Mutter 26 aufzuschrauben und diese so vom ersten Implantatteil 12 zu lösen.

Auch hier ist zu beachten, dass Abmessungen des Ausdrehwerkzeugs 166 auf die engen Platzverhältnisse aufgrund der im Durchmesser begrenzten Längsdurchbrechung 16 abgestimmt sind.

Die Außenteile 44 sowie die Innenteile 42 der Ausführungsbeispiele zwei bis fünf der Mutter 26, ebenso wie das Ausführungsbeispiel der Mutter 26, wie es in Verbindung mit den Figuren 23 bis 28 beschrieben wurde, können aus den selben Materialien ausgebildet sein, wie im Zusammenhang mit dem in den Figuren 3 bis 6 beschriebenen ersten Ausführungsbeispiel der Mutter 26.

Das Funktionsprinzip aller Ausführungsbeispiele medizinischer Muttern 26, wie sie oben beschrieben sind, ist identisch und beruht auf der Verformbarkeit des Außenteils 44 bei Erreichen eines Grenzdrehmoments in Anzugsdrehrichtung.

Alle beschriebenen Ausführungsbeispiele können zweiteilig ausgebildet sein und jeweils ein einziges Innenteil 42 und einziges Außenteil 44 umfassen.

Ferner sind bei allen Ausführungsbeispielen das Innengewinde 46 und die Innenteilverzahnung 54 axial versetzt angeordnet, das heißt überlappen nicht. Mit anderen Worten ist bei einer beliebigen Schnittansicht quer zur Längsachse 38 bei den Muttern 26 entweder ein Innengewinde 46 oder eine Innenteilverzahnung 54 sichtbar.

Mit den beschriebenen medizinischen Muttern 26 ist es möglich, insbesondere Implantatteile 12 und 14 ohne Einsatz eines Drehmomentschlüssels miteinander zu verbinden.

Die beschriebenen Muttern 26 können insbesondere auch dann noch einwandfrei wieder gelöst werden, wenn eine Verformung des Außenteils 44 auch teilweise plastisch erfolgen, also zu einer dauerhaften Verformung des Außenteils 44 führen sollte.

Beim Einsetzen der Wirkung der Drehmomentbegrenzungseinrichtung48 der Muttern 26 ist aufgrund der zurückschnappenden Außenteilzähne 60 bei Rückverformung des Außenteils 44 in eine unverformte Grundstellung ein Klicken zu hören, so dass ein Operateur beim Anziehen der Muttern 26 sowohl haptisch als auch akustisch eine Rückmeldung erhält, wenn das auf das Außenteil 44 eingeleitete Anzugsdrehmoment mindestens dem Grenzdrehmoment entspricht.

### Bezugszeichenliste

- 10: Implantat
- 12: erstes Implantatteil
- 14: zweites Implantatteil
- 16: Längsdurchbrechung
- 18: Kupplungsabschnitt
- 20: Schulter
- 22: Außengewindeabschnitt
- 24: Ende
- 26: Mutter
- 28: Innengewindeabschnitt
- 30: Außenmehrkant
- 32: Außensechskant
- 34: Klemmring
- 35: Ende
- 36: Außenfläche
- 38: Längsachse
- 40: Schraubverbindung
- 42: Innenteil
- 43: Ende
- 44: Außenteil
- 45: Außenteil
- 46: Innengewinde
- 48: Drehmomentbegrenzungseinrichtung
- 50: Pfeil
- 52: Außenhülse
- 54: Innenteilverzahnung
- 56: Außenteilverzahnung
- 58: Innenteilzahn
- 60: Außenteilzahn
- 62: Innenseite
- 64: distaler Hülsenabschnitt
- 66: proximaler Hülsenabschnitt
- 68: Verbindungsabschnitt
- 70: Anschlagfläche
- 72: Außenteillänge
- 74: Außenmehrkantlänge
- 76: Längsdurchbrechung
- 78: Innengewindelänge
- 80: Innenteillänge
- 82: Innenhülse
- 84: proximaler Hülsenabschnitt
- 86: mittlerer Hülsenabschnitt
- 88: distaler Hülsenabschnitt
- 90: Außenteilwand
- 92: Dicke
- 94: Pfeil
- 96: Innenteilzahnflanke
- 98: Außenteilzahnflanke
- 100: Schnittebene
- 102: Schnittebene
- 104: Außenteilzahnaufgleitflanke
- 106: Innenteilzahnaufgleitflanke
- 108: Abstand
- 109: Endabschnitt
- 110: Abstand
- 112: Pfeil
- 114: Übergangsbereich
- 116: Innenteilzahnspitze
- 118: Radialebene
- 120: Außenteilzahnspitze
- 122: Radialebene
- 124: Hinterschneidung
- 126: Hinterschneidung
- 128: Schlitz
- 130: Schlitz
- 132: Winkel
- 134: Randfläche
- 136: Ebene
- 138: Winkel
- 140: Flansch
- 142: Längsachse
- 144: Endfläche
- 146: Ringfläche
- 148: Außenkontur
- 150: Konuswinkel
- 152: Kante
- 154: Innenteilzahnaußenfläche
- 156: Übergangsbereich
- 158: Ende
- 160: Höhe
- 162: Gesamthöhe
- 164: Ende
- 166: Ausdrehwerkzeug
- 168: Innengewinde

## Patentansprüche

1. Implantat (10) mit einem ersten Implantatteil (12) und einem zweiten Implantatteil (14), wobei der erste und der zweite Implantatteil (12, 14) über eine eine Mutter (26) umfassende Schraubverbindung (40) in einer Verbindungsstellung miteinander verbunden sind, wobei die Mutter (26) in Form einer medizinischen Mutter (26) ausgebildet ist, welche eine Längsachse (38) definiert und ein Innengewinde und einen Außenmehrkant (30) aufweist, **dadurch gekennzeichnet, dass** die Mutter (26) mindestens ein Innenteil (42) und mindestens ein das Innenteil (42) mindestens teilweise, insbesondere vollständig oder im Wesentlichen vollständig, umgebendes Außenteil (44) umfasst, dass das mindestens eine Innenteil (42) das Innengewinde (46) umfasst, dass das mindestens eine Außenteil (44) den Außenmehrkant (30) umfasst, dass die Mutter (26) eine Drehmomentbegrenzungseinrichtung (48) umfasst, welche derart ausgebildet ist, dass bei einem in einer Anzugsdrehrichtung (50) auf das mindestens eine Außenteil (44) wirkenden Drehmoment, welches kleiner als ein Grenzdrehmoment ist, das mindestens eine Außenteil (44) und das mindestens eine Innenteil (42) in der Anzugsdrehrichtung (50) relativ zueinander unverdrehbar sind, dass bei einem in der Anzugsdrehrichtung (50) auf das mindestens eine Außenteil (44) wirkenden Drehmoment, welches mindestens dem Grenzdrehmoment entspricht, das mindestens eine Außenteil (44) und das mindestens eine Innenteil (42) in der Anzugsdrehrichtung (50) relativ zueinander verdrehbar sind, dass das mindestens eine Innenteil (42) und das mindestens eine Außenteil (44) unlösbar miteinander gekoppelt sind, dass die medizinische Mutter (26) zweiteilig ausgebildet ist und ausschließlich ein einziges Innenteil (42) und ein einziges Außenteil (44) umfasst oder dass die medizinische Mutter (26) dreiteilig ausgebildet ist und ausschließlich ein einziges Innenteil (42) und zwei Außenteile (44, 45) umfasst.

2. Implantat nach Anspruch 1, **dadurch gekennzeichnet, dass** das mindestens eine Außenteil (44) und/oder das mindestens eine Innenteil (42) temporär verformbar ausgebildet sind, so dass sich das mindestens eine Außenteil (44) und/oder das mindestens eine Innenteil (42) bei einem in der Anzugsdrehrichtung (50) auf das mindestens eine Außenteil (44) wirkenden Drehmoment, welches mindestens dem Grenzdrehmoment entspricht, derart verformen, dass das mindestens eine Außenteil (44) und das mindestens eine Innenteil (42) in der Anzugsdrehrichtung (50) relativ zueinander verdrehbar sind.

3. Implantat nach Anspruch 1 oder 2, **dadurch gekennzeichnet, dass** die Drehmomentbegrenzungseinrichtung (48) eine am mindestens einen Innenteil (42) angeordnete oder ausgebildete, von der Längsachse (38) in radialer Richtung weg weisende oder in axialer Richtung weisende Innenteilverzahnung (54) und eine am mindestens einen Außenteil (44) angeordnete oder ausgebildete, in radialer Richtung auf die Längsachse (38) hin weisende oder in axialer Richtung auf die Innenteilverzahnung weisende und mit der Innenteilverzahnung (54) zusammenwirkende Außenteilverzahnung (56) umfasst derart, dass bei einem in der Anzugsdrehrichtung (50) auf das mindestens eine Außenteil (44) wirkenden Drehmoment, welches kleiner als das Grenzdrehmoment ist, das mindestens eine Außenteil (44) und das mindestens eine Innenteil (42) in der Anzugsdrehrichtung (50) relativ zueinander unverdrehbar sind und dass bei einem in der Anzugsdrehrichtung (50) auf das mindestens eine Außenteil (44) wirkenden Drehmoment, welches mindestens dem Grenzdrehmoment entspricht, das mindestens eine Außenteil (44) und das mindestens eine Innenteil (42) in der Anzugsdrehrichtung relativ zueinander verdrehbar sind,
wobei insbesondere die Innenteilverzahnung (54) eine sich in distaler Richtung erweiternde konische Außenkontur (148) definiert, wobei insbesondere ein von der Außenkontur (148) definierter Konuswinkel (150) in einem Bereich von etwa 0° bis etwa 5° liegt, insbesondere 1,5° beträgt.

4. Implantat nach einem der voranstehenden Ansprüche, **dadurch gekennzeichnet, dass** das mindestens eine Innenteil (42) und das mindestens eine Außenteil (44) in einer Löserichtung (94) derart zusammenwirken, dass bei einer Verdrehung des mindestens einen Außenteils (44) um die Längsachse (38) in der der Anzugsdrehrichtung (50) entgegengesetzt gerichteten Lösedrehrichtung (94) das mindestens eine Innenteil (42) zusammen mit dem mindestens einen Außenteil (44) in der Lösedrehrichtung (94) verdreht wird,
wobei insbesondere
a) die Innenteilverzahnung (54) und die Außenteilverzahnung (56) bei Verdrehung des mindestens einen Außenteils (44) um die Längsachse (38) in der Lösedrehrichtung (94) ineinandergreifen und/oder
b) das mindestens eine Außenteil (44) und das mindestens eine Innenteil (42) in der Löserichtung (94) um die Längsachse (38) um einen Lösedrehwinkel von höchstens 360° relativ zueinander verdrehbar sind.

5. Implantat nach einem der voranstehenden Ansprüche, **dadurch gekennzeichnet, dass** das mindestens eine Außenteil (44) eine Außenteilwand (90) aufweist und dass eine Dicke (92) der Außenteilwand (90) über einen Umfang des mindestens einen Außenteils (44) variiert,
wobei insbesondere das mindestens eine Außenteil in der Außenteilwand (90) mindestens einen Schlitz (128) aufweist,
wobei weiter insbesondere
a) das Implantat eine Mehrzahl von Schlitzen (128) umfasst und/oder
b) der mindestens eine Schlitz (128) parallel oder schräg zur Längsachse verläuft.

6. Implantat nach einem der voranstehenden Ansprüche, **dadurch gekennzeichnet, dass**
a) das mindestens eine Außenteil (44) in Form einer Außenhülse (32) ausgebildet ist
und/oder
b) das mindestens eine Innenteil (42) in Form einer Innenhülse (82) ausgebildet ist
und/oder
c) das mindestens eine Außenteil (44) und das mindestens eine Innenteil (42) in der Anzugsdrehrichtung (50) um die Längsachse (38) um einen Anzugsdrehwinkel von höchstens 360° relativ zueinander verdrehbar sind, wenn das Anzugsdrehmoment kleiner als das Grenzdrehmoment ist,
und/oder
d) das mindestens eine Außenteil (44) distalseitig über das mindestens eine Innenteil (42) vorsteht
und/oder
e) das mindestens eine Innenteil (42) proximalseitig über das mindestens eine Außenteil (44) vorsteht.

7. Implantat nach einem der Ansprüche 3 bis 6, **dadurch gekennzeichnet, dass** die Innenteilverzahnung (54) mindestens einen Innenteilzahn (58) umfasst, insbesondere einen, zwei, drei, vier, fünf, sechs, sieben, acht oder neun Innenteilzähne (58), und dass die Außenteilverzahnung (56) mindestens einen Außenteilzahn umfasst, insbesondere einen, zwei, drei, vier, fünf, sechs, sieben, acht oder neun Außenteilzähne (60) wobei insbesondere
a) eine Anzahl der Schlitze (128) in der Außenteilwand (90) einer Anzahl der Außenteilzähne (60) entspricht
und/oder
b) in der Außenteilwand (90) Außenteilzähne (60) und Schlitze (128) gleichmäßig über einen Umfang verteilt, insbesondere alternierend, angeordnet oder ausgebildet sind
und/oder
c) die Dicke (92) der Außenteilwand (90) zwischen zwei Außenteilzähnen (60) ein Minimum aufweist
und/oder
d) sich der mindestens eine Innenteilzahn (58) parallel oder im Wesentlichen parallel zur Längsachse (38) erstreckt oder relativ zur Längsachse (38) um einen Neigungswinkel geneigt ist, insbesondere um einen Neigungswinkel in einem Bereich von etwa 0° bis etwa 10°,
und/oder
e) der mindestens eine Innenteilzahn (58) mindestens eine Innenteilzahnlöseflanke (96) aufweist, dass der mindestens eine Außenteilzahn (60) mindestens eine Außenteilzahnlöseflanke (98) aufweist und dass die Innenteilzahnlöseflanke (96) und die Außenteilzahnlöseflanke (98) in einer Lösestellung beim Verdrehen der Mutter (26) in der Löserichtung aneinander anliegen,
wobei insbesondere die Innenteilzahnlöseflanke (96) und/oder die Außenteilzahnlöseflanke (98) eine die Längsachse (38) enthaltende Schnittebene (100, 102) unter einem Winkel von maximal 30° schneiden
und/oder
die mindestens eine Innenteilzahnlöseflanke (96) bezogen auf eine die Längsachse und eine Innenteilzahnspitze (116) des mindestens einen Innenteilzahns (58) enthaltende Radialebene (118) hinterschnitten ist und/oder dass die mindestens eine Außenteilzahnlöseflanke (96) bezogen auf eine die Längsachse (38) und eine Außenteilzahnspitze des mindestens einen Außenteilzahns (60) enthaltende Radialebene (122) hinterschnitten ist.

8. Implantat nach Anspruch 7, **dadurch gekennzeichnet, dass** der mindestens eine Außenteilzahn (60) mindestens eine Außenteilzahnaufgleitflanke (104) aufweist, dass der mindestens eine Innenteilzahn (58) mindestens eine Innenteilzahnaufgleitflanke (106) aufweist, dass die mindestens einen Außenteilzahnaufgleitflanke (104) und die mindestens eine Innenteilzahnaufgleitflanke (106) aneinander klemmend gehalten sind, wenn das in der Anzugsdrehrichtung (50) auf das mindestens eine Außenteil (44) wirkende Drehmoment kleiner als das Grenzdrehmoment ist, und dass die mindestens einen Außenteilzahnaufgleitflanke (104) und die mindestens eine Innenteilzahnaufgleitflanke (106) aneinander entlanggleiten, wenn das in der Anzugsdrehrichtung (50) auf das mindestens eine Außenteil (44) wirkende Drehmoment mindestens dem Grenzdrehmoment entspricht,
wobei insbesondere
a) der mindestens eine Innenteilzahn (58) eine Innenteilzahnaußenfläche (154) definiert, dass die Innenteilzahnaußenfläche (154) und die Innenteilzahnaufgleitfläche (106) gegeneinander geneigt sind und in einem verrundeten Übergangsbereich (156) ineinander übergehen,
wobei insbesondere ein Radius des Übergangsbereichs (156) in einem Bereich von etwa 0,5 mm bis etwa 3 mm liegt, insbesondere beträgt der Radius 1,5 mm,
und/oder
b) die mindestens eine Außenteilzahnaufgleitflanke (104) flacher ist als die mindestens eine Außenteilzahnlöseflanke (98) und/oder dass die mindestens eine Innenteilzahnaufgleitflanke (106) flacher ist als die mindestens eine Innenteilzahnlöseflanke (96).

9. Implantat nach einem der voranstehenden Ansprüche, **dadurch gekennzeichnet, dass** das mindestens eine Innenteil (42) eine sich koaxial zur Längsachse (38) erstreckende Längsdurchbrechung (76) aufweist und dass die Längsdurchbrechung (76) mindestens einen das Innengewinde (46) bildenden Innengewindeabschnitt (28) umfasst,
wobei insbesondere das Innengewinde (46) eine Innengewindelänge (78) parallel zur Längsachse (38) aufweist, dass das mindestens eine Innenteil (42) einen Innenteillänge (80) parallel zur Längsachse (38) aufweist und dass die Innengewindelänge (78) mindestens etwa der halben Innenteillänge (80) entspricht.

10. Implantat nach einem der voranstehenden Ansprüche, **dadurch gekennzeichnet, dass**
a) der Außenmehrkant (30) eine Außenmehrkantlänge (74) parallel zur Längsachse (38) aufweist, dass das mindestens eine Außenteil (44) eine Außenteillänge (72) parallel zur Längsachse (38) aufweist und dass die Außenmehrkantlänge (74) höchstens etwa der halben Außenteillänge (72) entspricht
und/oder
b) das mindestens eine Innenteil (42) und das mindestens eine Außenteil (44) in axialer Richtung parallel zur Längsachse (38) unbeweglich oder im Wesentlichen unbeweglich miteinander gekoppelt sind
und/oder
c) das mindestens eine Innenteil (42) einstückig oder einteilig ausgebildet ist und/oder dass das mindestens eine Außenteil (44) einstückig oder einteilig ausgebildet ist.

11. Implantat nach einem der voranstehenden Ansprüche, **dadurch gekennzeichnet, dass** das mindestens eine Innenteil (42) aus einem Innenteilmaterial ausgebildet ist, dass das mindestens eine Außenteil (44) aus einem Außenteilmaterial ausgebildet ist und dass das Außenteilmaterial weicher und elastischer ist als das Innenteilmaterial.

12. Implantat nach Anspruch 11, **dadurch gekennzeichnet, dass** ein Elastizitätsmodul des Innenteilmaterials größer ist als ein Elastizitätsmodul des Außenteilmaterials.

13. Implantat nach Anspruch 11 oder 12, **dadurch gekennzeichnet, dass** das Innenteilmaterial und das Außenteilmaterial Metalle oder metallische Werkstoffe sind oder enthalten.

14. Implantat nach einem der Ansprüche 11 bis 13, **dadurch gekennzeichnet, dass**
a) das Innenteilmaterial ein Instrumentenstahl ist, welcher insbesondere Kobalt und/oder Chrom enthält, oder Nitinol oder Stahl und/oder
b) das Außenteilmaterial Titan enthält und/oder eine Titanlegierung ist, insbesondere Ti6Al4V.

15. Implantat nach einem der voranstehenden Ansprüche, **dadurch gekennzeichnet, dass**
a) die Mutter (26) ein Außenteil (45) in Form eines Klemmrings (34) umfasst, welcher distalseitig eine sich konisch verjüngende Außenfläche (36) aufweist, wobei insbesondere die Außenfläche (36) ein distales Ende der Mutter (26) definiert
und/oder
b) das Implantat (10) in Form eines Gelenkimplantats oder eines Teils eines Gelenkimplantats ausgebildet ist, insbesondere in Form eines Revisionsimplantats.

## Claims

1. Implant (10) having a first implant part (12) and a second implant part (14), wherein the first and the second implant part (12, 14) are connected to one another in a connecting position by way of a screw connection (40) comprising a nut (26), wherein the nut (26) is configured in the form of a medical nut (26), which defines a longitudinal axis (38) and has an internal thread and an external polygon (30), **characterized in that** the nut (26) comprises at least one inner part (42) and at least one outer part (44) at least partially, in particular completely or substantially completely, surrounding the inner part (42), **in that** the at least one inner part (42) comprises the internal thread (46), **in that** the at least one outer part (44) comprises the external polygon (30), **in that** the nut (26) comprises a torque limiting device (48), which is configured in such a way that the at least one outer part (44) and the at least one inner part (42) are non-rotatable relative to one another in a tightening direction (50) when a torque acting in the tightening direction (50) on the at least one outer part (44) is smaller than a limit torque, **in that** the at least one outer part (44) and the at least one inner part (42) are rotatable relative to one another in the tightening direction (50) when a torque acting in the tightening direction (50) on the at least one outer part (44) corresponds at least to the limit torque, **in that** the at least one inner part (42) and the at least one outer part (44) are non-releasably coupled to one another, **in that** the medical nut (26) is of two-part configuration and comprises only one single inner part (42) and one single outer part (44), or **in that** the medical nut (26) is of three-part configuration and comprises only one single inner part (42) and two outer parts (44, 45).

2. Implant in accordance with Claim 1, **characterized in that** the at least one outer part (44) and/or the at least one inner part (42) are configured to be temporarily deformable, such that the at least one outer part (44) and/or the at least one inner part (42) deform in such a way that the at least one outer part (44) and the at least one inner part (42) are rotatable relative to one another in the tightening direction (50) when a torque acting in the tightening direction (50) on the at least one outer part (44) corresponds at least to the limit torque.

3. Implant in accordance with Claim 1 or 2, **characterized in that** the torque limiting device (48) comprises an inner part toothing (54) that is arranged or formed on the at least one inner part (42) and points away from the longitudinal axis (38) in the radial direction or points in the axial direction and an outer part toothing (56) that is arranged or formed on the at least one outer part (44), points in the radial direction toward the longitudinal axis (38) or points in the axial direction toward inner part toothing, and cooperates with the inner part toothing (54) in such a way that the at least one outer part (44) and the at least one inner part (42) are non-rotatable relative to one another in the tightening direction (50) when a torque acting in the tightening direction (50) on the at least one outer part (44) is smaller than the limit torque and that the at least one outer part (44) and the at least one inner part (42) are rotatable relative to one another in the tightening direction when a torque acting in the tightening direction (50) on the at least one outer part (44) corresponds at least to the limit torque,
wherein, in particular, the inner part toothing (54) defines a conical outer contour (148) that widens in the distal direction, wherein, in particular, a cone angle (150) defined by the outer contour (148) is in a range of about 0° to about 5°, in particular is 1.5°.

4. Implant in accordance with any one of the preceding Claims, **characterized in that** the at least one inner part (42) and the at least one outer part (44) cooperate in a release direction (94) in such a way that the at least one inner part (42) is rotated together with the at least one outer part (44) in the release direction (94) upon a rotation of the at least one outer part (44) about the longitudinal axis (38) in the release direction (94) oppositely directed from the tightening direction (50), wherein, in particular,
a) the inner part toothing (54) and the outer part toothing (56) interengage upon rotation of the at least one outer part (44) about the longitudinal axis (38) in the release direction (94)
and/or
b) the at least one outer part (44) and the at least one inner part (42) are rotatable relative to one another in the release direction (94) about the longitudinal axis (38) by a release rotation angle of at most 360°.

5. Implant in accordance with any one of the preceding Claims, **characterized in that** the at least one outer part (44) has an outer part wall (90) and **in that** a thickness (92) of the outer part wall (90) varies over a periphery of the at least one outer part (44),
wherein, in particular, the at least one outer part has at least one slot (128) in the outer part wall (90),
wherein, further in particular,
a) the implant comprises a plurality of slots (128)
and/or
b) the at least one slot (128) extends in parallel or obliquely to the longitudinal axis.

6. Implant in accordance with any one of the preceding Claims, **characterized in that**
a) the at least one outer part (44) is configured in the form of an outer sleeve (32)
and/or
b) the at least one inner part (42) is configured in the form of an inner sleeve (82)
and/or
c) the at least one outer part (44) and the at least one inner part (42) are rotatable relative to one another in the tightening direction (50) about the longitudinal axis (38) by a tightening rotation angle of at most 360° when the tightening torque is smaller than the limit torque,
and/or
d) the at least one outer part (44) projects on the distal side over the at least one inner part (42)
and/or
e) the at least one inner part (42) projects on the proximal side over the at least one outer part (44).

7. Implant in accordance with any one of Claims 3 to 6, **characterized in that** the inner part toothing (54) comprises at least one inner part tooth (58), in particular one, two, three, four, five, six, seven, eight, or nine inner part teeth (58), and **in that** the outer part toothing (56) comprises at least one outer part tooth, in particular one, two, three, four, five, six, seven, eight, or nine outer part teeth (60)
wherein, in particular,
a) a number of slots (128) in the outer part wall (90) corresponds to a number of outer part teeth (60)
and/or
b) in the outer part wall (90), outer part teeth (60) and slots (128) are arranged or formed, in particular alternatingly, distributed uniformly over a periphery
and/or
c) the thickness (92) of the outer part wall (90) between two outer part teeth (60) has a minimum
and/or
d) the at least one inner part tooth (58) extends in parallel or substantially in parallel to the longitudinal axis (38) or is inclined relative to the longitudinal axis (38) by an angle of inclination, in particular by an angle of inclination in a range of about 0° to about 10°,
and/or
e) the at least one inner part tooth (58) has at least one inner part tooth release flank (96), **in that** the at least one outer part tooth (60) has at least one outer part tooth release flank (98), and **in that** the inner part tooth release flank (96) and the outer part tooth release flank (98) abut against one another in a release position when the nut (26) is rotated in the release direction,
wherein, in particular, the inner part tooth release flank (96) and/or the outer part tooth release flank (98) intersect a cut plane (100, 102) containing the longitudinal axis (38) at an angle of at most 30°
and/or
the at least one inner part tooth release flank (96) is undercut in relation to a radial plane (118) containing the longitudinal axis and an inner part tooth tip (116) of the at least one inner part tooth (58) and/or **in that** the at least one outer part tooth release flank (96) is undercut in relation to a radial plane (122) containing the longitudinal axis (38) and an outer part tooth tip of the at least one outer part tooth (60).

8. Implant in accordance with Claim 7, **characterized in that** the at least one outer part tooth (60) has at least one outer part tooth sliding flank (104), **in that** the at least one inner part tooth (58) has at least one inner part tooth sliding flank (106), **in that** the at least one outer part tooth sliding flank (104) and the at least one inner part tooth sliding flank (106) are held against one another in a clamping manner when the torque acting in the tightening direction (50) on the at least one outer part (44) is smaller than the limit torque, and **in that** the at least one outer part tooth sliding flank (104) and the at least one inner part tooth sliding flank (106) slide along one another when the torque acting in the tightening direction (50) on the at least one outer part (44) corresponds at least to the limit torque,
wherein, in particular,
a) the at least one inner part tooth (58) defines an inner part tooth outer surface (154), **in that** the inner part tooth outer surface (154) and the inner part tooth sliding surface (106) are inclined relative to one another and merge into one another in a rounded transition region (156), wherein, in particular, a radius of the transition region (156) is in a range of about 0.5 mm to about 3 mm, in particular the radius is 1.5 mm,
and/or
b) the at least one outer part tooth sliding flank (104) is flatter than the at least one outer part tooth release flank (98), and/or **in that** the at least one inner part tooth sliding flank (106) is flatter than the at least one inner part tooth release flank (96).

9. Implant in accordance with any one of the preceding Claims, **characterized in that**, **characterized in that** the at least one inner part (42) has a longitudinal perforation (76) extending coaxially to the longitudinal axis (38) and **in that** the longitudinal perforation (76) comprises at least one internal thread portion (28) forming the internal thread (46),
wherein, in particular, the internal thread (46) has an internal thread length (78) in parallel to the longitudinal axis (38), **in that** the at least one inner part (42) has an inner part length (80) in parallel to the longitudinal axis (38), and **in that** the internal thread length (78) corresponds at least to about half the inner part length (80).

10. Implant in accordance with any one of the preceding Claims, **characterized in that**
a) the external polygon (30) has an external polygon length (74) in parallel to the longitudinal axis (38), **in that** the at least one outer part (44) has an outer part length (72) in parallel to the longitudinal axis (38), and **in that** the external polygon length (74) corresponds at most to about half the outer part length (72)
and/or
b) the at least one inner part (42) and the at least one outer part (44) are coupled to one another so as to be immovable or substantially immovable in the axial direction in parallel to the longitudinal axis (38)
and/or
c) the at least one inner part (42) is of one-piece or one-part configuration, and/or **in that** the at least one outer part (44) is of one-piece or one-part configuration.

11. Implant in accordance with any one of the preceding Claims, **characterized in that** the at least one inner part (42) is made of an inner part material, **in that** the at least one outer part (44) is made of an outer part material, and **in that** the outer part material is softer and more elastic than the inner part material.

12. Implant in accordance with Claim 11, **characterized in that** a modulus of elasticity of the inner part material is greater than a modulus of elasticity of the outer part material.

13. Implant in accordance with Claim 11 or 12, **characterized in that** the inner part material and the outer part material are or contain metals or metallic materials.

14. Implant in accordance with any one of Claims 11 to 13, **characterized in that**
a) the inner part material is an instrument steel, which contains, in particular, cobalt and/or chromium, or Nitinol or steel
and/or
b) the outer part material contains titanium and/or a titanium alloy, in particular Ti6Al4V.

15. Implant in accordance with any one of the preceding Claims, **characterized in that**
a) the nut (26) comprises an outer part (45) in the form of a clamping ring (34), which has on the distal side a conically tapering outer surface (36), wherein, in particular, the outer surface (36) defines a distal end of the nut (26)
and/or
b) the implant (10) is configured in the form of a joint implant or a part of a joint implant, in particular in the form of a revision implant.

## Revendications

1. Implant (10) avec une première partie d'implant (12) et une seconde partie d'implant (14), où la première et la seconde partie d'implant (12, 14) sont liées l'une à l'autre dans une position de liaison par le biais d'une liaison à vis (40) comprenant un écrou (26), où l'écrou (26) est conçu sous la forme d'un écrou médical (26) qui définit un axe longitudinal (38) et présente un filetage interne et un polygone externe (30), **caractérisé en ce que** l'écrou (26) comprend au moins une partie interne (42) et au moins une partie externe (44) entourant au moins partiellement, en particulier complètement ou sensiblement complètement, la partie interne (42), **en ce que** la au moins une partie interne (42) comprend le filetage interne (46), **en ce que** la au moins une partie externe (44) comprend le polygone externe (30), **en ce que** l'écrou (26) comprend un dispositif limiteur de couple (48) qui est conçu de telle sorte que dans le cas d'un couple agissant sur la au moins une partie externe (44) dans un sens de rotation de serrage (50), qui est inférieur à un couple limite, la au moins une partie externe (44) et la au moins une partie interne (42) ne peuvent pas tourner l'une par rapport à l'autre dans le sens de rotation de serrage (50), **en ce que** dans le cas d'un couple agissant sur la au moins une partie externe (44) dans le sens de rotation de serrage (50) qui correspond au moins au couple limite, la au moins une partie externe (44) et la au moins une partie interne (42) peuvent tourner l'une par rapport à l'autre dans le sens de rotation de serrage (50), **en ce que** la au moins une partie interne (42) et la au moins une partie externe (44) sont couplées l'une à l'autre de manière permanente, **en ce que** l'écrou médical (26) est conçu en deux parties et comprend exclusivement une seule partie interne (42) et une seule partie externe (44) ou **en ce que** l'écrou médical (26) est conçu en trois parties et comprend exclusivement une seule partie interne (42) et deux parties externes (44, 45).

2. Implant selon la revendication 1, **caractérisé en ce que** la au moins une partie externe (44) et/ou la au moins une partie interne (42) sont conçues pour être temporairement déformables, de sorte que la au moins une partie externe (44) et/ou la au moins une partie interne (42), dans le cas d'un couple agissant sur la au moins une partie externe (44) dans le sens de rotation de serrage (50), qui correspond au moins au couple limite, se déforment de telle sorte que la au moins une partie externe (44) et la au moins une partie interne (42) peuvent tourner l'une par rapport à l'autre dans le sens de rotation de serrage (50).

3. Implant selon la revendication 1 ou 2, **caractérisé en ce que** le dispositif limiteur de couple (48) comprend une denture de partie interne (54) disposée ou conçue sur la au moins une partie interne (42), dirigée dans la direction radiale à l'opposé de l'axe longitudinal (38) ou dirigée dans la direction axiale et une denture de partie externe (56) disposée ou conçue sur la au moins une partie externe (44), dirigée dans la direction radiale vers l'axe longitudinal (38) ou dirigée dans la direction axiale vers la denture de partie interne et coopérant avec la denture de partie interne (54) de telle sorte que dans le cas d'un couple agissant sur la au moins une partie externe (44) dans le sens de rotation de serrage (50), qui est inférieur au couple limite, la au moins une partie externe (44) et la au moins une partie interne (42) ne peuvent pas tourner l'une par rapport à l'autre dans le sens de rotation de serrage (50) et **en ce que** dans le cas d'un couple agissant sur la au moins une partie externe (44) dans le sens de rotation de serrage (50), qui correspond au moins au couple limite, la au moins une partie externe (44) et la au moins une partie interne (42) peuvent tourner l'une par rapport à l'autre dans le sens de rotation de serrage,
où en particulier la denture de partie interne (54) définit un contour externe conique (148) qui s'élargit dans la direction distale, où en particulier un angle de cône (150) défini par le contour externe (148) est situé dans une plage d'environ 0° à environ 5°, en particulier est de 1,5°.

4. Implant selon l'une des revendications précédentes, **caractérisé en ce que** la au moins une partie interne (42) et la au moins une partie externe (44) coopèrent dans un sens de desserrage (94) de telle manière que lors d'une rotation de la au moins une partie externe (44) autour de l'axe longitudinal (38) dans le sens de rotation de desserrage (94) opposé au sens de rotation de serrage (50), la au moins une partie interne (42) tourne avec la au moins une partie externe (44) dans le sens de rotation de desserrage (94),
où en particulier
a) la denture de partie interne (54) et la denture de partie externe (56) s'engrènent lors de la rotation de la au moins une partie externe (44) autour de l'axe longitudinal (38) dans le sens de rotation de desserrage (94),
et/ou
b) la au moins une partie externe (44) et la au moins une partie interne (42) peuvent tourner l'une par rapport à l'autre autour de l'axe longitudinal (38) dans le sens de desserrage (94) d'un angle de rotation de desserrage d'au plus 360°.

5. Implant selon l'une des revendications précédentes, **caractérisé en ce que** la au moins une partie externe (44) présente une paroi de partie externe (90) et **en ce qu'**une épaisseur (92) de la paroi de partie externe (90) varie sur une circonférence de la au moins une partie externe (44),
où en particulier la au moins une partie externe présente dans la paroi de partie externe (90) au moins une fente (128),
où en particulier encore
a) l'implant comprend une pluralité de fentes (128) et/ou
b) la au moins une fente (128) s'étend parallèlement ou inclinée par rapport à l'axe longitudinal.

6. Implant selon l'une des revendications précédentes, **caractérisé en ce que**
a) la au moins une partie externe (44) est conçue sous la forme d'un manchon externe (32)
et/ou
b) la au moins une partie interne (42) est conçue sous la forme d'un manchon interne (82)
et/ou
c) la au moins une partie externe (44) et la au moins une partie interne (42) peuvent tourner l'une par rapport à l'autre dans le sens de rotation de serrage (50) autour de l'axe longitudinal (38) d'un angle de rotation de serrage d'au plus 360°, lorsque le couple de serrage est inférieur au couple limite,
et/ou
d) la au moins une partie externe (44) est en saillie du côté distal sur la au moins une partie interne (42)
et/ou
e) la au moins une partie interne (42) est en saillie du côté proximal sur la au moins une partie externe (44).

7. Implant selon l'une des revendications 3 à 6, **caractérisé en ce que** la denture de partie interne (54) comprend au moins une dent de partie interne (58), en particulier une, deux, trois, quatre, cinq, six, sept, huit ou neuf dents de partie interne (58), et **en ce que** la denture de partie externe (56) comprend au moins une dent de partie externe, en particulier une, deux, trois, quatre, cinq, six, sept, huit ou neuf dents de partie externe (60),
où en particulier
a) un nombre des fentes (128) dans la paroi de partie externe (90) correspond à un nombre des dents de partie externe (60)
et/ou
b) dans la paroi de partie externe (90) les dents de partie externe (60) et les fentes (128) sont agencées ou conçues régulièrement réparties sur une circonférence, en particulier en alternance,
et/ou
c) l'épaisseur (92) de la paroi de partie externe (90) présente un minimum entre deux dents de partie externe (60)
et/ou
d) la au moins une dent de partie interne (58) s'étend parallèlement ou sensiblement parallèlement à l'axe longitudinal (38) ou est inclinée par rapport à l'axe longitudinal (38) d'un angle d'inclinaison, en particulier d'un angle d'inclinaison dans une plage d'environ 0° à environ 10°,
et/ ou
e) la au moins une dent de partie interne (58) présente au moins un flanc de desserrage de dent de partie interne (96), **en ce que** la au moins une dent de partie externe (60) présente au moins un flanc de desserrage de dent de partie externe (98) et **en ce que** le flanc de desserrage de dent de partie interne (96) et le flanc de desserrage de dent de partie externe (98) sont adjacents dans une position de desserrage lors de la rotation de l'écrou (26) dans le sens de desserrage,
où en particulier le flanc de desserrage de dent de partie interne (96) et/ou le flanc de desserrage de dent de partie externe (98) coupent un plan de coupe (100, 102) contenant l'axe longitudinal (38) sous un angle d'au maximum 30°
et/ou
le au moins un flanc de desserrage de dent de partie interne (96) est en contre-dépouille par rapport à un plan radial (118) contenant l'axe longitudinal et une pointe de dent de partie interne (116) de la au moins une dent de partie interne (58) et/ou **en ce que** le au moins un flanc de desserrage de dent de partie externe (96) est en contre-dépouille par rapport à un plan radial (122) contenant l'axe longitudinal (38) et une pointe de dent de partie externe de la au moins une dent de partie externe (60).

8. Implant selon la revendication 7, **caractérisé en ce que** la au moins une dent de partie externe (60) présente au moins un flanc de glissement de dent de partie externe (104), **en ce que** la au moins une dent de partie interne (58) présente au moins un flanc de glissement de dent de partie interne (106), **en ce que** le au moins un flanc de glissement de dent de partie externe (104) et le au moins un flanc de glissement de dent de partie interne (106) sont maintenus l'un contre l'autre par serrage lorsque le couple agissant sur la au moins une partie externe (44) dans le sens de rotation de serrage (50) est inférieur au couple limite, et **en ce que** le au moins un flanc de glissement de dent de partie externe (104) et le au moins un flanc de glissement de dent de partie interne (106) glissent l'un contre l'autre lorsque le couple agissant sur la au moins une partie externe (44) dans le sens de rotation de serrage (50) correspond au moins au couple limite,
où en particulier
a) la au moins une dent de partie interne (58) définit une surface externe de dent de partie interne (154), **en ce que** la surface externe de dent de partie interne (154) et la surface de glissement de dent de partie interne (106) sont inclinées l'une par rapport à l'autre et se rejoignent dans une zone de transition arrondie (156),
où en particulier un rayon de la zone de transition (156) est situé dans une plage d'environ 0,5 mm à environ 3 mm, en particulier le rayon est de 1,5 mm,
et/ou
b) le au moins un flanc de glissement de dent de partie externe (104) est plus plat que le au moins un flanc de desserrage de dent de partie externe (98) et/ou **en ce que** le au moins un flanc de glissement de dent de partie interne (106) est plus plat que le au moins un flanc de desserrage de dent de partie interne (96).

9. Implant selon l'une des revendications précédentes, **caractérisé en ce que** la au moins une partie interne (42) présente une ouverture longitudinale (76) s'étendant coaxialement à l'axe longitudinal (38) et **en ce que** l'ouverture longitudinale (76) comprend au moins une section de filetage interne (28) formant le filetage interne (46),
où en particulier le filetage interne (46) présente une longueur de filetage interne (78) parallèle à l'axe longitudinal (38), **en ce que** la au moins une partie interne (42) présente une longueur de partie interne (80) parallèle à l'axe longitudinal (38) et **en ce que** la longueur de filetage interne (78) correspond à au moins environ la moitié de la longueur de partie interne (80).

10. Implant selon l'une des revendications précédentes, **caractérisé en ce que**
a) le polygone externe (30) présente une longueur de polygone externe (74) parallèle à l'axe longitudinal (38), **en ce que** la au moins une partie externe (44) présente une longueur de partie externe (72) parallèle à l'axe longitudinal (38) et **en ce que** la longueur de polygone externe (74) correspond au plus à environ la moitié de la longueur de partie externe (72)
et/ou
b) la au moins une partie interne (42) et la au moins une partie externe (44) sont couplées l'une à l'autre de manière fixe ou sensiblement fixe dans la direction axiale parallèle à l'axe longitudinal (38)
et/ou
c) la au moins une partie interne (42) est conçue monobloc ou d'une seule pièce et/ou **en ce que** la au moins une partie externe (44) est conçue monobloc ou d'une seule pièce.

11. Implant selon l'une des revendications précédentes, **caractérisé en ce que** la au moins une partie interne (42) est conçue en un matériau de partie interne, **en ce que** la au moins une partie externe (44) est conçue en un matériau de partie externe et **en ce que** le matériau de partie externe est plus souple et plus élastique que le matériau de partie interne.

12. Implant selon la revendication 11, **caractérisé en ce qu'**un module d'élasticité du matériau de partie interne est supérieur à un module d'élasticité du matériau de partie externe.

13. Implant selon la revendication 11 ou 12, **caractérisé en ce que** le matériau de partie interne et le matériau de partie externe sont ou contiennent des métaux ou des matières métalliques.

14. Implant selon l'une des revendications 11 à 13, **caractérisé en ce que**
a) le matériau de partie interne est un acier pour instruments, qui contient en particulier du cobalt et/ou du chrome, ou du nitinol ou de l'acier
et/ou
b) le matériau de partie externe contient du titane et/ou est un alliage de titane, en particulier Ti6Al4V.

15. Implant selon l'une des revendications précédentes, **caractérisé en ce que**
a) l'écrou (26) comprend une partie externe (45) sous la forme d'une bague de serrage (34), qui présente du côté distal une surface externe (36) qui diminue de manière conique, où en particulier la surface externe (36) définit une extrémité distale de l'écrou (26)
et/ou
b) l'implant (10) est conçu sous la forme d'un implant articulaire ou d'une partie d'un implant articulaire, en particulier sous la forme d'un implant pour révision.
